(19)

Europäisches
Patentamt
European
Patent Office
Office européen
des brevets

(11) **EP 2 832 360 A1**

(12) **EUROPEAN PATENT APPLICATION**

(43) Date of publication:
**04.02.2015 Bulletin 2015/06**

(51) Int Cl.:
*A61K 31/716* [(2006.01)]      *A61K 31/718* [(2006.01)]
*A61K 31/721* [(2006.01)]      *A61P 35/04* [(2006.01)]

(21) Application number: **13178566.9**

(22) Date of filing: **30.07.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Fresenius Kabi Deutschland GmbH
61352 Bad Homburg (DE)**

(72) Inventors:
• **Sundermann, Bernd
  61381 Friedrichsdorf (DE)**

• **Walz, Lars
  61440 Oberursel (DE)**
• **Baasner, Silke
  61137 Schöneck (DE)**
• **Nocken, Frank
  60320 Frankfurt am Main (DE)**
• **Meyer, Christoph
  61348 Bad Homburg v.d.H. (DE)**

(74) Representative: **Fresenius Kabi Deutschland
GmbH
Patent Department
Borkenberg 14
61440 Oberursel (DE)**

(54) **Polysaccharide for use in preventing metastasis formation and/or relapse**

(57)    The present invention relates to a polysaccharide comprising, optionally substituted, monosaccharide units linked via alpha-glycosidic bonds for use in preventing metastasis formation and/or relapse by administration to a body cavity of a subject afflicted with cancer. The present invention further relates to a method for preventing metastasis formation in a subject afflicted with cancer, comprising administering said composition to a body cavity of said subject, and thereby preventing metastasis formation in said subject. The present invention also relates to a composition and to a kit comprising a polysaccharide and a pharmaceutically acceptable means of solubilizing the same and to a device comprising a polysaccharide and means for administering the same.

EP 2 832 360 A1

**Description**

The present invention relates to a polysaccharide comprising, optionally substituted, monosaccharide units linked via alpha-glycosidic bonds for use in preventing metastasis formation and/or relapse by administration to a body cavity of a subject afflicted with cancer. The present invention further relates to a method for preventing metastasis formation in a subject afflicted with cancer, comprising administering said composition to a body cavity of said subject, and thereby preventing metastasis formation in said subject. The present invention also relates to a composition and to a kit comprising a polysaccharide and a pharmaceutically acceptable means of solubilizing the same and to a device comprising a polysaccharide and means for administering the same.

Polysaccharides derived from starch, have been used in medicine, e.g. as volume expanders in plasma substitution, but also in clinical hemodialysis (Sommermeyer et al., 1987, Krankenhauspharmazie, 8(8): 271-278; Weidler et al., 1991, Arzneimittelforschung/Drug Research, 41: 494-498). Frequently, specific forms of hydroxyalkylated starch (HAS), in particular hydroxyethylated starch (HES), are used for this purpose.

However, over the years, further potential medical uses of polysaccharides have been described:

ß-Glucans have been studied in oral and i.v. applications as global immune stimulants, in particular in cancer treatment (A. Weitberg, J Exp Clin Cancer Res (2008) 27: 40; WO2007/084661) and in a mouse sarkoma model (US 4,207,312). It was found that $\beta$-glucans have no direct cytotoxic effect, e.g. on cancer cells, but have immune-stimulatory effects (Chan et al., J Hematol Oncol (2009), 2: 25, WO2004/030613).

DE 40 23 788 Al describes the use of hydroxyalkyl starch for hyperbaric oxygen therapy of the inner ear. The only example describes the administration of a HES solution comprising an extract of Ginkgo biloba to a patient receiving hyperbaric treatment. There is, however, no indication of a therapeutic effect of such a treatment.

There are also several disclosures of the use of solutions comprising polyglucans as carriers for pharmaceutically active compounds: US 6,207,654 teaches the use of HES to prevent leakage of serum proteins from capillary endothelial junctions and proposes to use solutions comprising HES and interleukin-2 to treat viral and bacterial infection with the aim of preventing malignancy. Mohamed et al describe (EJSO (2003) 29:261) and review (Surg Oncol Clin N Am (2003) 12: 813) the advantages of isotonic high molecular weight solutions as carriers for intraperitoneal chemotherapy. Also, icodextrin has been described as a constituent of a carrier solution used in intraperitoneal adenoviral oncotherapy in a mouse model, showing that icodextrin solution improved overall survival as compared to PBS (Rocconi et al., Gynecologic Oncology (2006) 103: 985).

Moreover, polyglucans have been proposed for reducing postoperative adhesion formation, see, e.g. Van den Tol et al. (Surgery (2005) 137(3): 348), US 5,807,833, and I. Bekes (Dissertation an der Medizinischen Fakultät der RWTH Aachen (2008): "Adhäsions- und Nidationsprophylaxe nach i.p. Implantation von SCOV.ip-Zellen in SCID-Mäuse mittels Icodextrin, Hyaluronsäure und physiologischer NaCl-Lösung"). The latter document also examined the use of an icodextrin-solution for the prevention of nidation of tumor cells in lesions introduced into the abdominal cavity. No significant effect in comparison to the control (NaCl-solution) was found.

Cancer is still one of the major causes of death, especially in the developed countries. Accordingly, research on improved treatment and prevention of cancer is still the focus of many research projects. Over the years, methods have been devised for treating primary cancers which in most cases are at least initially effective. However, depending on the kind of cancer, there is a risk of regrowth of the cancer (relapse) and/or of spread of cancer cells to other sites of the body (metastasis). Accordingly, the five year survival rates vary from e.g. 100% for in situ breast cancer to 25% for ovary cancer and values as low as some 6% for pancreas cancer. Thus, in recent years, primary focus of research shifted from treatment of the primary cancer to prevention and treatment of metastasis and relapse. These topics appear especially demanding for the cancers of body cavities, especially the abdominal cavity; cancers growing in the abdominal cavity are cause symptoms only after they have reached a certain size, meaning that especially the risk of tumor cell detachment from the tumor, leading to metastasis, is much increased in those tumors. This effect contributes to the often abysmal prognosis of patient diagnosed with such cancers.

There is, thus, a need in the art to provide means and methods to prevent metastasis and relapse of tumors. The technical problem is solved by the embodiments characterized in the claims and herein below.

Accordingly, the present invention relates to a polysaccharide comprising, optionally substituted, monosaccharide units linked via alpha-glycosidic bonds for use in preventing metastasis formation and/or relapse by administration to a body cavity of a subject afflicted with cancer.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which a solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

Also, as used in the following, the term "about" in connection with a value of a parameter relates to said value including a range $\pm 10\%$, preferably $\pm 5\%$, more preferably $\pm 2\%$, most preferably $\pm 1\%$ of said value. Similarly, the term A "essentially consisting of" B relates to A consisting at least to a degree of 90%, preferably 95%, more preferably 98 %, most preferably 99% of B.

The term "polysaccharide", as used herein, relates to a polysaccharide comprising, optionally substituted, monosaccharide units linked via alpha-glycosidic bonds (alpha glycosidic bonds). The term alpha-glycosidic bond is known to those skilled in the art and relates to a covalent bond between a hemiacetal group of a saccharide and a hydroxyl group of a second molecule, preferably a saccharide. Accordingly, an "alpha-glycosidic bond" is a glycosidic bond fixing the anomeric carbon atom in the alpha position, e.g. in the case of D-glucopyranose in an axial orientation. Also, the term "glycosidically linked" relates to linkage via a glycosidic bond. It is understood by the skilled person that during formation of a glycosidic bond a molecule of water is released and that, therefore, e.g. a glucose unit engaged in a glycosidic bond is, preferably, related to as anhydroglucose.

Typically alpha-glycosidic bonds are more easily digested by the human body than beta-glycosidic bonds..

Methods for the analysis of polysaccharides and in particular, methods to determine the presence of alpha-glycosidic bonds are known to those skilled in the art. Preferably, the determination and/or the measurement is performed by IR and NMR analysis, such as 1 D and/or 2D NMR spectroscopy, in particular $^1$H-NMR, $^{13}$C-NMR, HSQC, TOCSY, COSY, NOESY and the like, according to standard protocols.

Preferably, the polysaccharide is a polysaccharide produced or producible by a living organism, or, more preferably, a derivative thereof. Most preferably, the polysaccharide is a polysaccharide produced by an organism from the kingdom plantae, in particular by a vascular plant (tracheophyte), or producible by an enzyme derived from a bacterium, or a derivative thereof.

Preferably, in the polysaccharide of the present invention, more than 90% of the monosaccharide units are linked via alpha-glycosidic bonds. More preferably, more than 95% of the monosaccharide units of the polysaccharide of the present invention are linked via alpha-glycosidic bonds. Most preferably, more than 98% of the monosaccharide units of the polysaccharide of the present invention are linked via alpha-glycosidic bonds.

The term "monosaccharide" is known to the skilled person. Preferably, the monosaccharide is a hexose, more preferably glucose. Most preferably, the monosaccharide is D-glucose. Preferably, the polysaccharide of the present invention may comprise more than one monosaccharide species. The monosaccharide units comprised in the polysaccharide of the present invention are substituted or unsubstituted as described herein below.

It is understood by the skilled person that a glycosidic bond between two glucose molecules may also be referred to as "glucosidic bond". Also, polysaccharides, in particular biopolymers, consisting of or essentially consisting of anhydroglucose units as monosaccharide units are referred to as "glucans". Accordingly, e.g., a polysaccharide consisting of or essentially consisting of anhydroglucose units connected via alpha-1,6-glycosidic bonds is referred to as alpha-(1,6)-Glucan; mutatis mutandis, a polysaccharide consisting of or essentially consisting of anhydroglucose units forming a backbone of alpha-1,4-glycosidically linked molecules together with branching points formed by alpha-1,6-glycosidic bonds would be referred to as an alpha-(1,4/1,6)-Glucan. It is also understood by the skilled person that the anhydroglucose units in a polysaccharide are often referred to as "glucose units" or as "glucose molecules" for simplicity.

In a preferred embodiment of the polysaccharide of the present invention, at least 70% of the monosaccharide units comprised in the polysaccharide are anhydroglucose and/or substituted anhydroglucose units. More preferably, at least 80% of the monosaccharide units comprised in the polysaccharide are anhydroglucose and/or substituted anhydroglucose units. Even more preferably, at least 90% of the monosaccharide units comprised in the polysaccharide are anhydroglucose and/or substituted anhydroglucose units. More preferably, at least 95%, more preferably at least 96 %, more preferably at least 97 %, more preferably at least 98 %, more preferably at least 99 %, of the monosaccharide units comprised in the polysaccharide are anhydroglucose and/or substituted anhydroglucose units. Accordingly, in a preferred embodiment, the polysaccharide of the present invention is a Glucan or a substituted Glucan.

Preferably the, optionally substituted, monosaccharide units, preferably the optionally substituted anhydroglucose units, are linked via alpha-1,4-glycosidic bonds and/or via alpha-1,6-glycosidic bonds and/or via alpha-1,3-glycosidic bonds and/or via 1,2-glycosidic bonds. Thus, preferably, the polysaccharide of the present invention is a polysaccharide comprising alpha-1,4-glycosidic bonds and/or alpha-1,6-glycosidic bonds and/or alpha-1,3-glycosidic bonds and/or 1,2 glycosidic bonds.

More preferably, the polysaccharide has a backbone chain consisting of alpha-1,4-glycosidically linked anhydroglucose units and/or alpha-1,6-glycosidically linked anhydroglucose units and/or alpha-1,3-glycosidically linked anhydroglucose units and/or alpha-1,2-glycosidically linked anhydroglucose units, preferably a backbone chain consisting of alpha-1,4-glycosidically linked anhydroglucose units and/or alpha-1,6-glycosidically linked anhydroglucose units and/or alpha-1,3-glycosidically linked anhydroglucose units.

According to one preferred embodiment, the polysaccharide has a backbone chain consisting of alpha-1,4-glycosidically linked anhydroglucose units. According to a further preferred embodiment, the polysaccharide has a backbone chain consisting of alpha-1,3-glycosidically linked anhydroglucose units. According to a further preferred embodiment, the polysaccharide has a backbone chain consisting of alpha-1,6-glycosidically linked anhydroglucose units. According to a further preferred embodiment, the polysaccharide has a backbone chain consisting of alpha-1,6-glycosidically linked anhydroglucose units and alpha-1,4-glycosidically linked anhydroglucose units. According to a further preferred embodiment, the polysaccharide has a backbone chain consisting of alpha-1,6-glycosidically linked anhydroglucose units and alpha-1,3-glycosidically linked anhydroglucose units. According to a further preferred embodiment, the polysaccharide has a backbone chain consisting of alpha-1,6-glycosidically linked anhydroglucose units and alpha-1,3-glycosidically linked anhydroglucose units and alpha-1,4-glycosidically linked anhydroglucose units.

The term "backbone chain" in this context refers to the main chain of the polysaccharide being formed of a series of covalently bonded building blocks, in particular alpha-1,4-glycosidically linked anhydroglucose units and/or alpha-1,6-glycosidically linked anhydroglucose units and/or alpha-1,3-glycosidically linked anhydroglucose units and/or alpha-1,2-glycosidically linked anhydroglucose units, that together create a continuous chain of the molecule. Preferably, the covalently connected chain of monosaccharide units providing the best structural continuity, i.e. the least variation in the kind of linkage, is regarded as the backbone chain. It is to be understood that the building blocks present in this chain may be unsubstituted or substituted, wherein the substitution pattern in each building block may be the same or may differ from each other. Further, a given monosacchride building block may be a branching point. Thus, the backbone chain may be substituted with at least one polysaccharide chain, yielding in a branched polysaccharide.

By way of example, without being limiting, the following Glucans are mentioned: an alpha-(1,4/1,6)-Glucan having an alpha-1,4 linked backbone together with alpha-1,6 linked branching points and preferably comprising an average about 1 alpha-1,6 linkage per 10 alpha-1,4 linkages; or, an alpha-(1,4/1,6)-Glucan having e.g. an alpha-1,4 linked backbone together with alpha-1,6 linked branching points and preferably comprising on average less than about 1 alpha-1,6 linkage per 10 alpha-1,4 linkages, or, an alpha-(1,4/1,6)-Glucan having an alpha-1,4 linked backbone together with alpha-1,6 linked branching points and preferably comprising on average about 1 alpha-1,6 linkage per 30 alpha-1,4 linkages.

It is understood by the skilled person that, in particular where the term "polysaccharide" relates to a biopolysaccharide, i.e. a polysaccharide produced or producible by (i.e. obtained from or obtainable from) a living organism, the polysaccharide as such may, comprise minor impurities, such as, e.g., in the backbone or in the side chains. The term "the polysaccharide has a backbone chain consisting of" thus also refers to polysaccharides comprising such minor impurities. Preferably, said minor impurities of the polysaccharide constitute less than 10% of the total mass of the polysaccharide, more preferably constitute less than 5% of the total mass of the polysaccharide, more preferably less than 4 %, more preferably less than 3 %, more preferably less than 2%, more preferably less than 1 %, more preferably less than 0.5 %, and, most preferably, constitute less than 0.1 % of the total mass of the polysaccharide.

Preferably, the polysaccharide does not comprise impurities in the form of beta-1,3-glycosidically linked anhydroglucose units, more preferably, the polysaccharide does not comprise impurities in the form of beta-glycosidically linked anhydroglucose units, most preferably, the polysaccharide does not comprise impurities in the form of β-glycosidically linked monosaccharide units.

According to a preferred embodiment, the polysaccharide comprises alpha-1,4-glycosidically linked, optionally substituted, anhydroglucose units.

According to a further preferred embodiment, the polysaccharide comprises alpha-1,6-glycosidically linked, optionally substituted, anhydroglucose units.

According to a further preferred embodiment, the polysaccharide comprises alpha-1,4-glycosidically linked and alpha-1,6-glycosidically linked, optionally substituted, anhydroglucose units.

According to a preferred embodiment, at least 90% of the glycosidic linkages of the polysaccharide are alpha-1,4-glycosidic linkages and/or alpha-1,6-glycosidic linkages. More preferably, at least 95%, more preferably at least 96 %, more preferably at least 97 %, more preferably at least 98 %, more preferably at least 99 %, of the glycosidic linkages of the polysaccharide are alpha-1,4-glycosidic linkages and/or alpha-1,6-glycosidic linkages.

Preferably, the polysaccharide of the present invention is thus an, optionally substituted, alpha-(1,6)-Glucan, optionally substituted, alpha-(1,4)-Glucan (e.g. maltodextrin), an, optionally substituted, alpha-(1,6/1,4)-Glucan (e.g.

pulllan), or an optionally substituted, alpha-(1,4/1,6)-Glucan having an alpha-1,4 linked backbone together with alpha-1,6 linked branching points.

The polysaccharide of the present invention may be a basic or an acidic polysaccharide, a zwitterionic or a neutral polysaccharide. Preferably, the polysaccharide polysaccharide is a neutral, i.e., preferably, an uncharged polysaccharide.

Preferably, the polysaccharide of the present invention has a number-average molecular weight, shortly referred to as "average molecular weight" herein, of at least 1 kDa, more preferably at least 5 kDa, more preferably at least 10 kDa, more preferably at least 13 kDa. Preferably, the polysaccharide has an average molecular weight of at most 1200 kDa, more preferably at most 1000 kDa, most preferably at most 800 kDa. Preferably, the polysaccharide has an average molecular weight in the range of from 5 to 1200 kDa, more preferably of from 10 to 1000 kDa, more preferably of from 13 to 800 kDa. Most preferred molecular weights are also shown herein below and in the Examples. Methods of determining the molecular weight of polysaccharides are known in the art. Mw and Mn of the polysaccharides of the present invention generally are determined according to the following method: The "mean molecular weight" as used in the context of the present invention relates to the weight as determined according to MALLS-GPC (Multiple Angle Laser Light Scattering-Gel Permeation Chromatography). For the determination, 2 Tosoh BioSep GMPWXL columns connected in line (13 $\mu$m particle size, diameter 7.8 mm, length 30 cm, Art.no. 08025) were used as stationary phase. The mobile phase was prepared as follows: In a volumetric flask 3.74 g Na-Acetate*3H2O, 0.344 g NaN3 are dissolved in 800 ml Milli-Q water and 6.9 ml acetic acid anhydride are added and the flask filled up to 1 1. Approximately 10 mg of the polysaccahride were dissolved in 1 ml of the mobile phase and particle filtrated with a syringe filter (0.22 mm, mStarII, CoStar Cambridge, MA) The measurement was carried out at a Flow rate of 0.5 ml/min. As detectors a multiple-angle laser light scattering detector and a refractometer maintained at a constant temperature, connected in series, were used. Astra software (Vers. 5.3.4.14, Wyatt Technology Cooperation) was used to determine the mean Mw and the mean Mn of the sample using a dn/dc of 0.147. The value was determined at $\lambda$=690 nm (solvent NaOAc/H2O/0.02%NaN3, T=20°C) in accordance to the following literature: W.M. Kulicke, U. Kaiser, D. Schwengers, R. Lemmes, Starch, Vol. 43, Issue 10 (1991), 392-396 and as described in WO 2012/004007 A1, Example 1.9. However, Mw and Mn values of HAS and HES related to in this specification are values determined according to the method of Sommermeyer et al. (loc. cit).

Preferably, the polysaccharide is selected from the group consisting of, unsubstituted amylopectin, substituted, amylopectin, unsubstituted starch, substituted starch, unsubstituted maltodextrin, unsubstituted dextran, unsubstituted icodextrin, substituted maltodextrin, substituted dextran, and substituted icodextrin. More preferably, the polysaccharide is selected from the group consisting of (optionally substituted) dextran, icodextrin, maltodextrin and hydroxyalkyl starch, more preferably, the polysaccharide is selected from the group consisting of dextran, icodextrin, and hydroxyalkyl starch.

In particular, the polysaccharide is not hyaluronic acid, preferably not a polysaccharide comprising glucosamine or a derivative thereof.

According to a preferred embodiment, the present invention relates to a polysaccharide for use in preventing metastasis formation and/or relapse by administration to a body cavity of a subject afflicted with cancer, wherein the polysaccharide comprises substituted or unsubstituted, alpha-1,4-glycosidically linked and/or alpha-1,6-glycosidically linked anhydroglucose units as described above. More preferably, the polysaccharide comprises, essentially consists of, alpha-1,4-glycosidically linked and/or alpha-1,6-glycosidically linked substituted or unsusbtituted anhydroglucose units. Even more preferably, the polysaccharide is a dextran or an icodextrin including substituted derivates thereof.

The term "dextran" is known to the skilled person and relates to a glucan typically comprising a backbone of alpha-1,6-glycosidically linked anhydroglucose units together with alpha-1,4- and alpha-1,3- and optionally alpha-1,2 linkages as branch points. Natural dextrans have molecular weights in a range between 10 and 50,000 kDa. Preferred dextrans are dextrans with molecular weights in a range between 10 and 10,000 kDa, more preferably between 10 and 1,000 kDa, and, most preferably, between 35 and 75 kDa.

Preferably, a composition comprising dextran as sole polysaccharide is used for preoperative, postoperative or intraoperative administration as detailed herein below.

The term "icodextrin" is also known to the skilled person and relates to a glucan typically comprising a backbone of alpha-1,4-glycosidically linked anhydroglucose units together with alpha-1,6- linkages as branch points. Preferred icodextrins have an Mw of 5 to 30 kDa, more preferably 10 to 20 kDa, and, most preferably, 13 to 16 kDa. Preferred icodextrins have an Mn of 3 to 10 kDa, more preferably of 4 to 7.5 kDa, and, most preferably, of 5 to 6 kDa. Accordingly, in a preferred embodiment, the icodextrin has an Mw of 13 to 16 kDa and an Mn of 5 to 6 kDa.

Preferably, a composition comprising icodextrin as sole polysaccharide is used for preoperative administration as detailed herein below.

According to an even more preferred embodiment, the present invention relates to a polysaccharide for use in preventing metastasis formation and/or relapse by administration to a body cavity of a subject afflicted with cancer, wherein the polysaccharide comprises at least one substituted anhydroglucose unit, wherein the at least one anhydroglucose unit is preferably substituted with at least one substituent selected from the group consisting of glycosyl, alkyl, cycloalkyl, aryl, halogen, amino acid and -(alkyl-)$_n$-H with n = 1-6, preferably 1-3, and wherein the alkyl group in each repeating unit n may be the same or may differ from each other, and wherein in each group -(alkyl-O)$_n$-H present in the polysaccharide, n may be the same or may differ from each other. It is to be understood that in case the hydroxyl groups of the anhydroglucose unit are substituted, a group "-O-Substituent " is formed. Alternatively, a hydroxyl group may be replaced with the respective substituent. Thus, the term "substituted anhydroglucose unit" refers to anydroglucose units, in which a substituent may be attached to the oxygen of the hydroxyl group thereby forming a group -O-substituent or may be directly attached to a carbon atom of the saccharide unit.

The term "substituted anhydroglucose" or "substituted monosacccharide" or "substituted polysacccharide", as used hereinabove and below, is known to the skilled person and relates to an anhydroglucose unit or a monosaccaride comprised in a polysaccharide according to the present invention modified by chemical modification in, preferably, at least one chemical modification reaction. The term "chemical modification reaction" is known to the skilled person and relates to a chemical reaction modifying the chemical structure of a polysaccharide or an anhydroglucose or monosaccharide unit comprised therein according to the present invention, preferably without changing its characteristic structural features. Thus, preferably, the term chemical modification reaction relates to partial hydrolysis or to a modification of a side chain of the polysaccharide of the present invention. Preferably, said modification of a side chain increases the half-life of the polysaccharide of the present invention at the site of administration. Preferably, modification of a side chain is alkylation, e.g.. methylation or ethylation, acylation, more preferably acetylation, glycosylation, hydroxylation, hydroxyalkylation, deacylation or demethylation, or derivatization with a piperazine, piperidine, piperidinamine, teneraic acid, piperidinepropanol, halogen, amino acid, or polypeptide, preferably olipopeptide, functional group, or any combination thereof. More preferably, said modification of a side chain is alkylation, even more preferably methylation or, most preferably, ethylation. Preferably, the derivative has the same or a similar activity with regard to the diseases referred to herein as the parent polysaccharide as described herein above and below. Preferably, the definition of the term "substituted" applies to substitution of other monosaccharide units mutatis mutandis. Preferably, modification of a side chain is modification with compound being free of anti-cancer activity.

As used herein, the term "alkyl group" is understood to comprise a linear or branched functional group or side-chain that consists of saturated hydrocarbons, preferably of a chain length of 2 to 12 carbon atoms. Said saturated hydrocarbon can be linear, such as propyl-, butyl-, pentyl-, hexyl-, heptyl-, octyl-, nonyl-, decanyl-, undecanyl- and dodecanyl-residues; or branched, i.e. wherein the carbon backbone splits off in one or more directions, comprising for example isopropyl-, isobutyl-, tert.-butyl, 1-isopentyl, 2-isopentyl, 3-isopentyl-, neopentyl-rests.

Preferably, the polysaccharide comprises at least one substituted monosaccharide unit, in particular, an anhydroglucose unit being substituted with at least one group -(alkyl-O)$_n$-H with n = 1-6, preferably 1-3, and wherein the alkyl group in each repeating unit n may be the same or may differ from each other, and wherein in each group -(alkyl-O)$_n$-H present in the polysaccharide, n may be the same or may differ from each other. In particular, the polysaccharide comprises -O-(alkyl-O)$_n$-H groups, in which the proton of the hydroxyl group is being substituted with the group -(alkyl-O)$_n$-H. Integer n is preferably of from 1 to 6, more preferably of from 1 to 4, such as 1, 2, 3 or 4, wherein, if more than one group - (alkyl-O)$_n$-H is present, n may be the same or may differ from each other, and the alkyl may be the same and may differ from each other, preferably in each group, the alkyl is the same.

Preferably, the polysaccharide comprises at least one substituted anhydroglucose unit being substituted with at least one group -(ethyl-O)$_n$-H, preferably at least one substituted anhydroglucose unit in which the proton of at least one hydroxyl group is being replaced with a group -(ethyl-O)$_n$-H (thereby forming -O-(ethyl-O)$_n$-H) with n = 1-6, preferably 1-3, and wherein the ethyl group in each repeating unit n may be the same or may differ from each other, and wherein in each group -(ethyl-O)$_n$-H present in the polysaccharide, n may be the same or may differ from each other.

In a particularly preferred embodiment, the polysaccharide of the present invention is hydroxyalkyl starch (HAS). As hydroxyalkyl starches, hydroxypropyl starch and hydroxyethyl starch are preferred, with hydroxyethyl starch being most preferred.

Preferably, a composition comprising HAS as sole polysaccharide is used for preoperative, postoperative or intraoperative administration as detailed herein below

Starch is a well-known polysaccharide according to formula $(C_6H_{10}O_5)_n$ which essentially consists of alpha-D glucose units which are coupled via glycosidic linkages. Usually, starch essentially consists of amylose and amylopectin. Amylose consists of linear chains wherein the glucose units are linked via alpha-1,4-glycosidic linkages. Amylopectin is a highly branched structure with alpha-1,4-glycosidic linkages and alpha-1,6-glycosidic linkages. Native starches from which hydroxyalkyl starches can be prepared include, but are not limited to, cereal starches and potato starches. Cereal starches include, but are not limited to, rice starches, wheat starches such as einkorn starches, spelt starches, soft wheat starches, emmer starches, durum wheat starches, or kamut starches, corn starches, rye starches, oat starches, barley starches, triticale starches, spelt starches, and millet starches such as sorghum starches or teff starches. Preferred native starches from which hydroxyalkyl starches are prepared have a high content of amylopectin relative to amylose. The amylopectin content of these starches is, for example, at least 70 % by weight, preferably at least 75 % by weight, more preferably at least 80 % by weight, more preferably at least 85 % by weight, more preferably at least 90 % by weight such as up to 95 % by weight, up to 96 % by weight, up to 97 % by weight, up to 98 % by weight, up to 99 % by weight, or up to 100 % by weight. Native starches having an especially high amylopectin content are, for example, suitable potato starches such as waxy potato starches which are preferably extracted from essentially amylose-free potatoes which are either traditionally bred (for example the natural variety Eliane) or genetically modified amylopectin potato varieties, and starches of waxy varieties of cereals such as waxy corn or waxy rice.

Hydroxyalkyl starch (HAS) is an ether derivative of partially hydrolyzed natural starches, in which hydroxyl groups in the starch are suitably hydroxyalkylated; thus, HAS comprises -O-(alkyl-O)$_n$-H groups, in which the proton of at least one hydroxyl group is being replaced with the group -(alkyl-O)$_n$-H with n being of from 1 to 6, preferably of from 1 to 4, more preferably of from 1 to 2, more preferably 1. As a polymer , and owing to the preparation processes, HAS is a polydisperse compound in which the individual hydroxyalkyl starch molecules may differ with respect to the degree of polymerization, the number and the pattern of the branching sites, and the substitution pattern, i.e. the number and/or sites of the hydroxyalkyl groups. Therefore, hydroxyalkyl starch is usually characterized by statistically averaged parameters. These are, generally, molecular weight distribution, the degree of substitution and the ratio of C2/C6 substitution.

There are two possibilities of describing the substitution degree. The degree of substitution (DS) of hydroxyalkyl starch is described relatively to the portion of substituted glucose monomers with respect to all glucose moieties. The substitution pattern of hydroxyalkyl starch can also be described as the molar substitution (MS), wherein the number of hydroxyalkyl groups per glucose moiety is counted. In the context of the present invention, the substitution pattern of hydroxyalkyl starch is described in terms of MS. Regarding MS, reference is also made to Sommermeyer et al., 1987, Krankenhauspharmazie, 8(8), 271-278, in particular p. 273. MS is determined by gas chromatography after total hydrolysis of the hydroxyalkyl starch. MS values of the respective hydroxyalkyl starch starting material are given. It is assumed that the MS value is not affected during the method according to the present invention.

Also, a particular hydroxyalkyl starch solution is, preferably, defined by the average molecular weight with the help of statistical means. In this context, $M_n$ or Mn is calculated as the arithmetic mean depending on the number of molecules and their molecular weight. The number average molecular weight $M_n$ is defined by the following equation:

$$M_n = \Sigma_i\, n_i M_i\, /\, \Sigma_i\, n_i$$

wherein $n_i$ is the number of hydroxyalkyl starch molecules of species i having molar mass $M_i$. Alternatively, the mass distribution can be described by the weight average molecular weight $M_w$ or Mw. The weight average molecular $M_w$ weight is defined by the following equation:

$$M_w = \Sigma_i\, n_i M_i^2\, /\, \Sigma_i\, n_i M_i$$

wherein $n_i$ is the number of hydroxyalkyl starch molecules of species i having molar mass $M_i$. According to the present invention, $M_w$ values are preferably in the range of from 1 to 2000 kDa, more preferably of from 5 to 700 kDa, more preferably of from 10 to 300 kDa, more preferably of from 70 to 150 kDa.

It is understood by the skilled person that the average molecular weight may be determined according to Sommermeyer et al. (Krankenhauspharmazie, 8, 1987, 08, p. 271-278) or according to European Pharmacopoeia 7.0, 01/2011:1785, p.984. The difference between the two methods is the value of the light scattering value dn/dc used: in the Sommermeyer method, a dn/dc value of 0.135 is used, whereas this value was changed to 0.147+/-0.001 in the Pharmacopoeia method. If not otherwise noted, values of average molecular weights as used herein relate to values as determined with the Sommermeyer method (loc. cit.).

The second parameter which is usually referred to as MS (molecular substitution) describes the number of hydroxyalkylated sites per anhydroglucose unit of a given hydroxyalkyl starch and may be determined according to Sommermeyer et al. (Krankenhauspharmazie 8 (8), 1987, pp 271-278, in particular page 273) or according to European Pharmacopoeia 7.0, 01/2011:1785, p.984. The values of MS correspond to the degradability of the hydroxyalkyl starch by alpha-amylase. Generally, the higher the MS value of the hydroxyalkyl starch, the lower is its respective degradability. The parameter MS can also be determined according to Ying-Che Lee et al., Anal. Chem. 55, 1983, pp 334-338; or K. L. Hodges et al., Anal. Chem. 51, 1979, p 2171. According to these methods, a known amount of the hydroxyalkyl starch is subjected to ether cleavage in xylene whereby adipinic acid and hydriodic acid are added. The amount of released iodoalkane is subsequently determined via gas chromatography using toluene as an internal standard and iodoalkane calibration solutions as external standards. According to the present invention, MS values are preferably in the range of from 0.1 to 3, more preferably from 0.2 to 1.3, more preferable from 0.3 to 0.7.

The third parameter which is referred to as "C2/C6 ratio" describes the ratio of the number of the anhydroglucose units being substituted in C2 position relative to the number of the anhydroglucose units being substituted in C6 position. During the preparation of the hydroxyalkyl starch, the C2/C6 ratio can be influenced via the pH used for the hydroxyalkylation reaction. Generally, the higher the pH, the more hydroxyl groups in C6 position are hydroxyalkylated. The parameter C2/C6 ratio can be determined, for example, according to Sommermeyer et al., Krankenhauspharmazie 8 (8), 1987, pp 271-278, in particular page 273. According to the present invention, typical values of the C2/C6 ratio are in the range of from 2 to 20, preferably of from 2 to 14, more preferably of from 2 to 12.

For practical reasons, the following nomenclature in the identification of different HAS and HES preparations is applied: An abbreviation letter code indicates the kind of modification (e.g. "HES" for hydroxyethyl starch), followed by two numbers, indicating the average molecular weight and the molecular substitution, respectively. Accordingly, "HES 130/0.4" indicates hydroxyethyl starch with an average molecular weight of 130 kDa and an MS of 0.4. It is understood by the skilled person that, since partial hydrolysis as well as substitution of side chains are statistical processes, the values indicated are average values including a certain range. Preferably, the MS values, and the C2/C6 values indicate a range of values $\pm$ 20%, more preferably $\pm$ 10%, most preferably $\pm$ 5%.

Accordingly, preferred embodiments of the polysaccharide of the present invention are HES 70/0.5, HES 130/0.4, and HES 450/0.7. Said specific HES derivatives are especially preferred for preoperative, intraoperative, and postoperative administration.

Concerning the preparation of hydroxyalkyl starch, more particularly of hydroxyethyl starch, reference is made, for example, to Sommermeyer et al., Chromatographia, 25, 1988, pp. 167-168; C. Jungheinrich et al., Clin. Pharmacokin., 44 (7), 2005, pp. 681-699; J.-M. Mishler IV, Pharmacology of hydroxyethyl starches, Oxford Medical Publications, 20 2002, pp. 1-30.

Preferably, the polysaccharide is osmotically active. As used herein, the term "osmotically active" relates a compound having the property of causing osmosis to occur. Thus, the osmotically active polysaccharide of the present invention, when present in a solution on one side of a semipermeable membrane, causes distilled water present on the other side to diffuse over said semipermeable membrane. Preferably, the property of a polysaccharide being osmotically active is determined by determining the osmolality of a 5% solution of the polysaccharide according to the standard method described in British Pharmacopoeia Volume V, Appendix V: N. Osmolality (2012). A polysaccharide having an osmolality of 100 mOsmol/kg as determined by the aforesaid method is considered to be osmotically active.

Preferably the polysaccharide is biodegradable, most preferably the polysaccharide is osmotically active and biodegradable.

The term "biodegradable", as used herein, relates to the property of the polysaccharide of the present invention of being capable of being decomposed through the action of a living organism within a specific time frame. Preferably, the term relates to the property of the polysaccharide of the present invention of being capable of being decomposed through the action of the metabolism of the subject said polysaccharide is administered to. More preferably, the term relates to the property of the polysaccharide of the present invention of being capable of being decomposed through the action of cells and/or enzymes present in the subject at the site of administration. Preferably, the time frame required for degradation of half of the amount of polysaccharide initially administered is at least one day, more preferably at least two days, most preferably at least three days. Preferably, the time frame required for degradation of half of the amount of polysaccharide initially administered is at most two months, more preferably at most one month, most preferably at most two weeks. Accordingly, the half life of the biodegradable polysaccharide according to the present invention at the site of administration and/or within the subject said polysaccharide is applied to, preferably is one day to two months, more preferably is two days to one month, and most preferably is three days to two weeks. The term "being decomposed", as used herein, relates to any process diminishing and/or terminating the osmotic activity of the biodegradable polysaccharide polysaccharide at the site of administration. Thus, it is understood by the skilled person that the term "being decomposed", preferably, relates to a complete decomposition or to a partial decomposition of the biodegradable polysaccharide of the present invention, or to a removal of the biodegradable polysaccharide from the site of administration. Preferably, the biodegradable polysaccharide is decomposed to an extent that permits metabolization and/or excretion of the degradation products by the subject of the present invention. From the above, it is, however, understood that the term "being decomposed", preferably, also includes taking up of the polysaccharide by cells present at and/or migrating to the site of administration in the absence of any decomposition of the polysaccharide molecules. Preferably, biodegradability is determined according to the method of Bekes (loc. cit) by determining polymeric glucose in a sample and comparing the amount determined to the amount administered or to a sample drawn earlier. It is understood by the skilled person that polysaccharides of the present invention comprising anhydroglucose units linked via alpha-glycosidic bonds, are biodegradable by alpha-Amylases.

The term "preventing", as used herein, refers to retaining health with respect to the diseases or disorders referred to herein for a certain period of time in a subject. It will be understood that the said period of time is dependent on the amount of the composition which has been administered and on individual factors of the subject discussed elsewhere in this specification. It is to be understood that prevention may not be effective in all subjects treated with the composition according to the present invention. However, the term requires that a, preferably statistically significant, portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

"Cancer", in the context of the present invention, refers to a disease of an animal, including man, characterized by uncontrolled growth by a group of body cells ("cancer cells"). This uncontrolled growth may be accompanied by intrusion into and destruction of surrounding tissue and possibly spread of cancer cells to other locations in the body (metastasis). Preferably, a metastasis is a metastasis of one of the cancers defined herein below, more preferably of a preferred cancer. It is known to the skilled person that a cancer may reappear after an initially successful treatment (relapse). Preferably, a relapse is a relapse of cancer in a subject after treatment with an anti-cancer drug or/and radiotherapy. More preferably, a relapse is a relapse of cancer in a subject, wherein the cancer cells of the relapse are resistant to an anti-cancer drug or/and radiotherapy. Preferably, relapse is a relapse of one of the cancers defined herein below, more preferably of a preferred cancer.

The term "cancer", as used herein, preferably refers to a proliferative disorder or disease caused or characterized by the proliferation of cells which have lost susceptibility to normal growth control ("cancer cells"). This uncontrolled growth may be accompanied by intrusion into and destruction of surrounding tissue and possibly spread of cancer cells to other locations in the body (metastasis). It is known to the skilled person that a cancer forming a solid tumor may reappear after an initially successful treatment (relapse). Preferably, the term encompasses tumors and any other proliferative disorders. Thus, the term is meant to include all pathological conditions involving malignant cells, irrespective of stage or of invasiveness. The term, preferably, includes solid tumors arising in solid tissues or organs as well as hematopoietic tumors (e.g. leukemias and lymphomas).

The cancer may be localized to a specific tissue or organ (e.g. in the ovar, the prostate or the pancreas), and, thus, may not have spread beyond the tissue of origin. Furthermore the cancer may be invasive, and, thus may have spread beyond the layer of tissue in which it originated into the normal surrounding tissues (frequently also referred to as locally advanced cancer). Invasive cancers may or may not be metastatic. Thus, the cancer may be also metastatic. A cancer is metastatic, if it has spread from its original location to distant parts of the body. E.g., it is well known in the art that breast cancer cells may spread to another organ or body part, such as the lymph nodes.

Preferably, the cancer is selected from the list consisting of Acute Lymphoblastic Leukemia (adult), Acute Lymphoblastic Leukemia (childhood), Acute Myeloid Leukemia (adult), Acute Myeloid Leukemia (childhood), Adrenocortical Carcinoma, Adrenocortical Carcinoma (childhood), AIDS-Related Cancers, AIDS-Related Lymphoma, Anal Cancer, Appendix Cancer, Astrocytomas (childhood), Atypical Teratoid/Rhabdoid Tumor (childhood), Central Nervous System Cancer, Basal Cell Carcinoma, Bile Duct Cancer (Extrahepatic), Bladder Cancer, Bladder Cancer (childhood), Bone Cancer, Osteosarcoma and Malignant Fibrous Histiocytoma, Brain Stem Glioma (childhood), Brain Tumor (adult), Brain Tumor (childhood), Brain Stem Glioma (childhood), Central Nervous System Brain Tumor, Atypical Teratoid/Rhabdoid Tumor (childhood), Brain Tumor, Central Nervous System Embryonal Tumors (childhood), Astrocytomas (childhood) Brain Tumor, Craniopharyngioma, Brain Tumor (childhood), Ependymoblastoma Brain Tumor (childhood), Ependymoma Brain Tumor (childhood), Medulloblastoma Brain Tumor (childhood), Medulloepitheliom Brain Tumor (childhood), Pineal Parenchymal Tumors of Intermediate Differentiation, Brain Tumor (childhood), Supratentorial Primitive Neuroectodermal Tumors and Pineoblastoma Brain Tumor, (childhood), Brain and Spinal Cord Tumors (childhood), Breast Cancer , Breast Cancer (childhood), Breast Cancer (Male), Bronchial Tumors (childhood), Burkitt Lymphoma, Carcinoid Tumor (childhood), Carcinoid Tumor, Gastrointestinal Carcinoma, Atypical Teratoid/Rhabdoid Tumor (childhood), Central Nervous System Embryonal Tumors (childhood), Central Nervous System (CNS) Lymphoma, Primary Cervical Cancer, Cervical Cancer (childhood), Childhood Cancers, Chordoma (childhood), Chronic Lymphocytic Leukemia, Chronic Myelogenous Leukemia, Chronic Myeloproliferative Disorders, Colon Cancer, Colorectal Cancer (childhood), Craniopharyngioma (childhood), Cutaneous T-Cell Lymphoma, Embryonal Tumors, Central Nervous System (childhod), Endometrial Cancer, Ependymoblastoma (childhood), Ependymoma (childhood), Esophageal Cancer, Esophageal Cancer (childhood), Esthesioneuroblastoma (childhood), Ewing Sarcoma Family of Tumors, Extracranial Germ Cell Tumor (childhood), Extragonadal Germ Cell Tumor, Extrahepatic Bile Duct Cancer, Eye Cancer, Intraocular Melanoma, Eye Cancer, Retinoblastoma, Gallbladder Cancer, Gastric (Stomach) Cancer, Gastric (Stomach) Cancer (childhood), Gastrointestinal Carcinoid Tumor, Gastrointestinal Stromal Tumor (GIST), Gastrointestinal Stromal Cell Tumor (childhood), Germ Cell Tumor, Extracranial (childhood), Germ Cell Tumor, Extragonadal, Germ Cell Tumor, Ovarian cancer, Gestational Trophoblastic Tumor, Glioma (adult), Glioma (childhood), Brain Stem cancer, Hairy Cell Leukemia, Head and Neck Cancer, Heart Cancer (childhood), Hepatocellular (Liver) Cancer (adult) (Primary), Hepatocellular (Liver) Cancer (childhood) (Primary), Histiocytosis, Langerhans Cell, Hodgkin Lymphoma (adult), Hodgkin Lymphoma (childhood), Hypopharyngeal Cancer, Intraocular Melanoma, Islet Cell Tumors (Endocrine Pancreas), Kaposi Sarcoma, Kidney (Renal Cell) Cancer, Kidney Cancer (childhood), Langerhans Cell Histiocytosis, Laryngeal Cancer, Laryngeal Cancer (childhood), Leukemia, Acute Lymphoblastic (adult), Leukemia, Acute Lymphoblastic (childhood), Leukemia, Acute Myeloid (adult), Leukemia, Acute Myeloid (childhood), Leukemia, Chronic Lymphocytic, Leukemia, Chronic Myelogenous, Leukemia, Hairy Cell, Lip and Oral Cavity Cancer, Liver Cancer (adult) (Primary), Liver Cancer (childhood) (Primary), Non-Small Cell Lung Cancer, Small Cell Lung Cancer, Non-Hodgkin Lymphoma, (adult), Non-Hodgkin Lymphoma, (childhood), Primary Central Nervous System (CNS) Lymphoma, Waldenström, Macroglobulinemia, Malignant Fibrous Histiocytoma of Bone and Osteosarcoma, Medulloblastoma (childhood), Medulloepithelioma (childhood), Melanoma, Intraocular (Eye)Melanoma, Merkel Cell Carcinoma, Mesothelioma (adult) Malignant, Mesothelioma (childhood), Metastatic Squamous Neck Cancer with Occult Primary, Mouth Cancer, Multiple Endocrine Neoplasia Syndromes (childhood), Multiple Myeloma/Plasma Cell Neoplasm, Mycosis Fungoides, Myelodysplastic Syndromes, Myelodysplastic/Myeloproliferative Neoplasms, Myelogenous Leukemia, Chronic, Myeloid Leukemia (adult) Acute, Myeloid Leukemia (childhood) Acute, Myeloma, Multiple, Nasal Cavity and Paranasal Sinus Cancer, Nasopharyngeal Cancer, Nasopharyngeal Cancer (childhood), Neuroblastoma, Oral Cancer (childhood), Lip and Oral Cavity Cancer, Oropharyngeal Cancer, Osteosarcoma and Malignant Fibrous, Histiocytoma of Bone, Ovarian Cancer (childhood), Ovarian Epithelial Cancer, Ovarian Germ Cell Tumor, Ovarian Low Malignant Potential Tumor, Pancreatic Cancer, Pancreatic Cancer (childhood), Pancreatic Cancer, Islet Cell Tumors, Papillomatosis (childhood), Paranasal Sinus and Nasal Cavity Cancer, Parathyroid Cancer, Penile Cancer, Pharyngeal Cancer, Pineal Parenchymal Tumors of Intermediate Differentiation (childhood), Pineoblastoma and Supratentorial Primitive Neuroectodermal Tumors (childhood), Pituitary Tumor, Plasma Cell Neoplasm/Multiple Myeloma, Pleuropulmonary Blastoma, Pregnancy and Breast Cancer, Primary Central Nervous System (CNS) Lymphoma, Prostate Cancer, Rectal Cancer, Renal Cell (Kidney) Cancer, Renal Pelvis and Ureter Transitional Cell Cancer, Respiratory Tract Cancer with Chromosome 15 Changes, Retinoblastoma, Rhabdomyosarcoma (childhood), Salivary Gland Cancer, Salivary Gland Cancer (childhood), Sarcoma, Ewing Sarcoma Family of Tumors, Kaposi Sarcoma, Soft Tissue (adult)Sarcoma, Soft Tissue (childhood)Sarcoma, Uterine Sarcoma, Sézary Syndrome, Skin Cancer (Nonmelanoma), Skin Cancer (childhood), Skin Cancer (Melanoma), Merkel Cell Skin Carcinoma, Small Cell Lung Cancer, Small Intestine Cancer, Soft Tissue Sarcoma (adult), Soft Tissue Sarcoma (childhood), Squamous Cell Carcinoma, see Skin Cancer (Nonmelanoma), Stomach (Gastric) Cancer, Stomach (Gastric) Cancer (childhood), Supratentorial Primitive Neuroectodermal Tumors (childhood), Cutaneous T-Cell Lymphoma, Testicular Cancer, Testicular Cancer (childhood), Throat Cancer, Thymoma and Thymic Carcinoma, Thymoma and Thymic Carcinoma (childhood), Thyroid Cancer, Thyroid Cancer (childhood), Transitional Cell Cancer of the Renal Pelvis and Ureter, T Gestational rophoblastic Tumor, Unknown Primary Site, Carcinoma of adult, Unknown Primary Site, Cancer of (childhood), Unusual Cancers of childhood, Ureter and Renal Pelvis, Transitional Cell Cancer, Urethral Cancer, Uterine Cancer, Endometrial, Uterine Sarcoma, Vaginal Cancer, Vaginal Cancer (childhood), Vulvar Cancer, Waldenström Macroglobulinemia and Wilms Tumor.

More preferably, the cancer is a cancer forming a tumor. Even more preferably, the cancer is ovarian cancer, ovarian carcinoma, stomach cancer, lung cancer, pancreas cancer, bladder cancer, or liver cancer. Most preferably, the cancer is colorectal cancer or breast cancer.

The term "body cavity", as used herein, relates to any hollow space within the body of a subject which may be filled with liquid, gas, and/or organs or parts thereof, including, e.g. the bladder. Preferably, the body cavity is a body cavity lined with a serous membrane, e.g. more preferably, an abdominal cavity, a pleural cavity, a synovial cavity, or a pericardial cavity. Most preferably, the body cavity is the abdominal cavity.

The term "subject" relates to an animal, preferably a mammal, more preferably a human. The term "subject afflicted with cancer" relates to a subject comprising and/or having comprised cancer cells, preferably a tumor, in its body. Preferably, the term relates to a subject for which it is known that it comprises and/or comprised cancer cells; thus, more preferably, the subject afflicted with cancer is a subject diagnosed to suffer from cancer or known to have suffered from cancer.

According to the present invention, the term "administration" relates to application of the composition or polysaccharide according to the present invention to a subject. Preferably, the term relates to a continuous administration. More preferably, the term relates to a repeated or to a one-time application. Preferably, administration relates to administration to a body cavity, more preferably to the abdominal cavity. The composition or the polysaccharide may be administered by any suitable method known to those skilled in the art. The preferred mode of administration depends on whether the composition or the polysaccharide is to be administered preoperative, intraoperative, and/or postoperative administration, i.e. whether the composition or the polysaccharide is to be administered to an open body cavity (which has been opened by surgical intervention, thus intraoperatively) or to a closed body cavity, i.e. preoperativly and/or postoperatively. Suitable administration methods are known to those skilled in the art and include, e.g. injection or infusion, in particular intraperitoneal injection or intraperitoneal infusion.

The term "surgery", as used herein, relates to a surgical intervention, preferably in a body cavity of a subject. It is understood that the term, preferably, relates to any kind of surgical intervention, irrespective whether the surgery is performed in the context of the subject's afflictedness with cancer. More preferably, the surgery of the present invention is a surgical intervention partially or, more preferably, completely removing a tumor (tumor resection) and/or a metastasis (metastasis resection), or a surgical intervention for obtaining a biopsy of a tumor and/or a metastasis.

The term "composition", as used herein, relates to a composition comprising or consisting of the polysaccharide as specified herein below. Preferably, the composition is a pharmaceutical composition. Preferably, the composition consists of the polysaccharide of the present invention.

According to a further preferred embodiment, the composition comprises further ingredients, more preferably pharmaceutically acceptable ingredients as known to the skilled person and as specified, by way of example, herein below. The term "other ingredients" relates. e.g., to at least one solvent as specified herein below or to other pharmaceutical acceptable additives and/or excipients.

The polysaccharide of the present invention can be formulated as pharmaceutically acceptable salt. Acceptable salts comprise acetate, methylester, HCl, sulfate, chloride and the like.

Preferred pharmaceutically acceptable ingredients are excipients, preferably selected from the list consisting of monosaccharides, disaccharides, inorganic salts, antimicrobial agents, antioxidants, surfactants, buffers, acids, bases, and any combination thereof.

Preferred monosaccharides are saccharides, such as fructose, maltose, galactose, glucose, D-mannose, sorbose, and the like; as disaccharides, lactose, sucrose, trehalose, cellobiose, and the like, are mentioned by way of example.

Preferred inorganic salts or buffers are citric acid, sodium chloride, potassium chloride, sodium sulfate, potassium nitrate, sodium phosphate monobasic, sodium phosphate dibasic, and any combination thereof. Preferred antimicrobial agents for preventing or detecting microbial growth are benzalkonium chloride, benzethonium chloride, benzyl alcohol, cetylpyridinium chloride, chlorobutanol, phenol, phenylethyl alcohol, phenylmercuric nitrate, thimersol, and any combination thereof. Preferred antioxidants are ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and any combination thereof. Preferred surfactants are polysorbates, or pluronics sorbitan esters; lipids, such as phospholipids and lecithin and other phosphatidylcholines, phosphatidylethanolamines, acids and fatty esters; steroids, such as cholesterol; and chelating agents, such as EDTA or zinc, and any compatible combination thereof. Preferred acids and bases are hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof. Other preferred pharmaceutically acceptable ingredients include vitamins, micronutrients, antioxidants, and the like.

Preferably, the "other ingredients" are galenic ingredients, i.e. ingredients not mediating a parmaceutical effect related to cancer cells. Thus, preferably, the composition comprises a polysaccharide of the present invention as the sole ingredient preventing metastasis formation and/or relapse.

More preferably, the composition comprises a polysaccharide of the present invention as the sole European Medicines Agency (EMA)- and/or Food and Drug Administration (FDA)-approved anti-cancer compound.

Most preferably, the composition comprises a polysaccharide of the present invention as the sole European Medicines Agency (EMA)- and/or Food and Drug Administration (FDA)-approved therapeutically active compound.

In a preferred embodiment, the polysaccharide is preferably dextran or HAS, more preferably HAS, most preferably HES, or the composition of the present invention preferably comprises dextran or HAS, more preferably HAS, most preferably HES, and the polysaccharide or the composition is administered postoperatively. Thus, the present invention also relates to a polysaccharide and to a composition comprising a polysaccharide, for use in preventing metastasis formation and/or relapse by administration to a body cavity of a subject afflicted with cancer, wherein the polysaccharide or the composition is to be administered postoperatively, and wherein the polysaccharide is preferably dextran or HAS, more preferably HAS, most preferably HES. The term "postoperative administration" relates to an administration after a surgical intervention as defined above was performed. Preferably, the term relates to a time frame between immediately after surgery and four weeks thereafter. More preferably, the term relates to a time frame between immediately after surgery and one week thereafter, even more preferably, the term relates to a time frame between immediately after surgery and 24 hours thereafter; most preferably, the term relates to a time frame between immediately after surgery and four hours thereafter. "After surgery" refers to the time after completion of the surgery, i.e. after closure of the previously formed incision, e.g. by sutures or staples. Preferably, "after surgery" refers to the closure of an incision performed at an organ, but before closing the body cavity. In each case, the polysaccharide or composition of the invention is preferably administered by injection, more preferably by intraperitoneal injection or intraperitoneal infusion.

In a further preferred embodiment, the polysaccharide is preferably dextran or HAS, more preferably HAS, most preferably HES, or the composition of the present invention preferably comprises dextran or HAS, more preferably HAS, most preferably HES, and the polysaccharide or the composition is administered intaoperatively. As is understood by the skilled person, the term "intraoperative administration" relates to an administration during a surgical intervention, i.e. before the closure of the incision. Thus, the present invention also relates to a polysaccharide and to a composition comprising a polysaccharide, for use in preventing metastasis formation and/or relapse by administration to a body cavity of a subject afflicted with cancer, wherein the polysaccharide or the composition is to be administered intraoperatively, and wherein the polysaccharide is dextran or HAS, more preferably HAS, most preferably HES.

In a further preferred embodiment, the polysaccharide is preferably icodextrin, dextran or HAS, more preferably dextran or HAS; more preferably HAS, most preferably HES, or the composition of the present invention preferably comprises icodextrin, dextran or HAS, more preferably dextran or HAS; more preferably HAS, most preferably HES, and the polysaccharide or the composition is administered preoperatively. The term "preoperative administration" relates to an administration in a time frame between four weeks and immediately before surgery. More preferably, the term relates to a time frame between three weeks and immediately before surgery. Even more preferably, the term relates to a time frame between two weeks and immediately before surgery. Most preferably, the term relates to a time frame between one week and immediately before surgery. It is understood that, preferably, administration of the polysaccharide to a subject will not start before the diagnosis that said subject is afflicted with cancer has been obtained. Thus, preferably, preoperative administration is administration during the time frame between cancer diagnosis and surgery as defined herein above. Thus, the present invention also relates to a polysaccharide and to a composition comprising a polysaccharide, for use in preventing metastasis formation and/or relapse by administration to a body cavity of a subject afflicted with cancer, wherein the polysaccharide or the composition is to be administered preoperatively, and wherein the polysaccharide is icodextrin, dextran or HAS, more preferably dextran or HAS; more preferably HAS, most preferably HES. In this case, the polysaccharide or composition of the invention is preferably administered by injection, more preferably by intraperitoneal injection or intraperitoneal infusion.

Preferably, the composition is a pharmaceutically acceptable solution. The term "pharmaceutically acceptable solution" as used herein relates to a composition comprising the polysaccharide of the present invention and one or more pharmaceutically acceptable liquid carrier(s).

The composition of the present invention, more preferably the pharmaceutically acceptable solution is, preferably, administered into a body cavity of a subject as defined herein above. More preferably, the pharmaceutically acceptable solution is administered to the abdominal cavity. The term "administration to a body cavity" is understood by the skilled person and relates to an administration to the lumen within the body cavity.

The liquid carrier(s) must be acceptable in the sense of being compatible with the ingredients of the formulation and being not deleterious to the recipient thereof. The liquid carrier(s) is/are selected so as not to affect the biological activity of the polysaccharide. Accordingly, the liquid carrier preferably is an isotonic or mildly hypo- or hypertonic solution of non-deleterious ingredients in a suitable solvent. Preferably, the suitable solvent comprises water, more preferably distilled water; thus, the pharmaceutically acceptable solution, preferably, is an aqueous solution. More preferably, the liquid carrier is physiological saline solution, phosphate buffered saline solution, cardioplegic solution, Ringer's solution, or Hank's solution. In addition, the pharmaceutically acceptable solution preferably includes other carriers or nontoxic, nontherapeutic, nonimmunogenic stabilizers and the like.

Preferably, the concentration of the polysaccharide in the pharmacologically acceptable solution is 0.5% (w/v) to 25% (w/v), more preferably 2% (w/v) to 15% (w/v), even more preferably 3% (w/v) to 12.5% (w/v), and most preferably 4% (w/v) to 10% (w/v), based on the total volume of the solution. Preferred concentrations and concentration ranges for specific embodiments of the present invention are the following: Preferably, the concentration of HAS, in particular HES, in the pharmacologically acceptable solution is 0.5% (w/v) to 25% (w/v), more preferably 2% (w/v) to 15% (w/v), even more preferably 3% (w/v) to 12.5% (w/v), and most preferably 4% (w/v) to 10% (w/v), based on the total volume of the solution. Preferably, the concentration of dextran in the pharmacologically acceptable solution is 0.5% (w/v) to 25% (w/v), more preferably 3% (w/v) to 20% (w/v), even more preferably 5% (w/v) to 15% (w/v), and most preferably 7.5% (w/v) to 12.5% (w/v), based on the total volume of the solution. Preferably, the concentration of icodextrin in the pharmacologically acceptable solution is 0.5% (w/v) to 25% (w/v), more preferably 1% (w/v) to 15% (w/v), even more preferably 2% (w/v) to 12.5% (w/v), and most preferably 3% (w/v) to 6% (w/v), based on the total volume of the solution.

Preferred pharmaceutically acceptable solutions comprising the polysaccharide of the present invention are, by way of example: HES 70/0.5, 60 g/L in physiological saline (0.9%), HES 130/0.4, 100 g/L in physiological saline (0.9%), and HES 450/0.7, 60 g/L in cardioplegic perfusion solution (magnesium-DL-hydrogenaspartate 4 $H_2O$ 0.721 g/L, Procainhydrochlorid 1.091 g/L, calcium chloride 2 $H_2O$ 0.074 g/L, sodium chloride 1.461 g/L, potassium chloride 0.373 g/L, glucose-monohydrate 1.982 g/L, mannitol 36.44 g/L). Said solutions are especially preferred for preoperative, intraoperative, and postoperative administration.

Another preferred pharmaceutically acceptable solution comprising the polysaccharide of the present invention is icodextrin, 40 g/L in an aqueous solution of sodium chloride 5.4 g/L, sodium lactate 4.5 g/L, calcium chloride 257 mg/L, magnesium chloride 61 mg/L, which is particularly preferred for preoperative administration.

Advantageously, it was found during the work underlying the present invention that the polysaccharides of the present invention, when applied to the abdominal cavity of a mammal, interfere with the nidation of cancer cells in the abdominal cavity. Even more surprisingly, it was found that the inhibitory effect is also observable in the absence of lesions caused, e.g. by surgical trauma, in the abdomen. This means that the polysaccharides are useful in the prevention of metastasis and/or relapse at any time the danger of nidation of free cancer cells, which may arise by detachment from a primary tumor, a metastasis, or a relapse, in a body cavity exists.

The definitions made above apply *mutatis mutandis* to the following. Additional definitions and explanations made further below also apply for all embodiments described in this specification *mutatis mutandis*.

The present invention also relates to a use of a polysaccharide of the present invention for the manufacture of a pharmaceutical preparation for preventing metastasis formation in a subject afflicted with cancer.

The present invention further relates to method for preventing metastasis formation in a body cavity of a subject afflicted with cancer, comprising a) administering a pharmaceutically acceptable solution comprising a polysaccharide to a body cavity of said subject, and b) thereby preventing metastasis formation in a body cavity of said subject.

The method of the present invention, preferably, is an in vivo method. Moreover, it may comprise steps in addition to those explicitly mentioned above. Moreover, one or more of said steps may be performed by automated equipment. For example, further treatment steps may relate, e.g., to identifying a subject as being afflicted with cancer before step a) or removing said pharmaceutically acceptable solution comprising a polysaccharide from said body cavity after step a) in combination with repeating step a), i.e. flushing said body cavity with said pharmaceutically acceptable solution comprising a polysaccharide.

Preferably, surgical removal of cancer cells is performed before, during, or after the step of administering a pharmaceutically acceptable solution comprising a polysaccharide to a body cavity of said subject. More preferably, surgical removal of cancer cells is, in a case wherein the cancer forms a solid tumor, removal of at least part of the primary tumor of said cancer before or after administering said aqueous solution comprising a polysaccharide in step a). More preferably, at least the primary tumor or a part thereof is removed by surgery in such case. Most preferably, at least the primary tumor is removed completely in such case.

Preferably, the amount of pharmaceutically acceptable solution comprising a polysaccharide administered is determined by the size and/or the capacity of the body cavity into which said solution is to be administered. It is understood by the skilled person that, in principle, it is preferable to administer a high volume of the pharmaceutically acceptable solution comprising a polysaccharide. However, it is also understood that the volume to be administered is naturally limited by the capacity of the body cavity and also that an effective dose of a polysaccharide can be administered by using a smaller volume of solution comprising a higher concentration of said polysaccharide.

An effective dose of the polysaccharide of the present invention is a dose which prevents metastasis and/or relapse in a subject. Efficacy and toxicity of compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors, preferably in accordance with any one of the above described methods. As is well known in the medical arts, a dosage for any one patient depends upon many factors, including the patient's size, body surface area, age, the particular compound to be administered, sex, time and route of administration, general health, and other drugs being administered concurrently. Efficacy can be monitored by periodic assessment. A typical dose can be, for example, in the range of 5 to 250 g of polysaccharide per administration into the abdominal cavity; however, doses below or above this exemplary range are envisioned, especially considering the aforementioned factors and considering the smaller size of other body cavities, e.g. the pericard. It envisioned that, preferably, the dose is adjusted accordingly. Generally, the regimen as a regular administration of the pharmaceutical composition should be in the range of 5 to 250 g per 4 to 7 days.

The pharmaceutically acceptable solution comprising a polysaccharide referred to herein is administered at least once in order to prevent a disease or condition recited in this specification. However, the said pharmaceutically acceptable solution comprising a polysaccharide may be administered more than one time, for example every four to seven days for up to several weeks.

It is understood that other modi of administration described herein apply with respect to the method for preventing metastasis formation in a body cavity as well.

Also, the present invention relates to a kit comprising a polysaccharide and a pharmacologically acceptable means of solubilizing the same.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that, preferably, the kit of the present invention is to be used for practicing the methods referred to herein above. It is, more preferably, envisaged that all components are provided in a ready-to-use manner for practicing the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention.

Further, the present invention relates to a device comprising a polysaccharide and means for administering the same.

The term "device", as used herein, relates to a system of means comprising at least the polysaccharide according the present invention referred to in the claims or herein, preferably comprised in a pharmaceutically acceptable solution, and a means of administering the same to a subject. Means of applying the polysaccharide of the present invention, including a pharmaceutically acceptable solution comprising the same, are well known to the skilled person and include, e.g. syringes, infusion sets, inhalers, and the like. Preferably, the aforesaid means are comprised by a single device.

By way of example, without being limiting, the following preferred embodiments are mentioned:

1. A polysaccharide comprising, optionally substituted, monosaccharide units linked via alpha-glycosidic bonds for use in preventing metastasis formation and/or relapse by administration to a body cavity of a subject afflicted with cancer.

2. The polysaccharide for use of embodiment 1, wherein the polysaccharide is for postoperative administration, for intraoperative administration, and/or for preoperative administration.

3. The polysaccharide for use of embodiment 1 or 2, wherein the polysaccharide comprises alpha-1,4-glycosidic bonds and/or alpha-1,6-glycosidic bonds and/or alpha-1,3-glycosidic bonds and/or 1,2 glycosidic bonds.

4. The polysaccharide for use of any one of embodiments 1 to 3, wherein at least 95%, more preferably at least 96 %, more preferably at least 97 %, more preferably at least 98 %, more preferably at least 99 %, of the monosaccharide units comprised in the polysaccharide are anhydroglucose and/or substituted anhydroglucose units.

5. The polysaccharide for use of any one of embodiments 1 to 4, wherein the polysaccharide comprises alpha-1,6-glycosidically linked anhydroglucose units.

6. The polysaccharide for use of any one of embodiments 1 to 4, wherein the polysaccharide comprises alpha-1,4-glycosidically linked, optionally substituted anhydroglucose units and alpha-1,6-glycosidically linked, optionally substituted anhydroglucose units.

7. The polysaccharide for use of any one of embodiments 1 to 6, wherein the polysaccharide is linear or branched.

8. The polysaccharide for use of any one of embodiments 1 to 7, wherein the polysaccharide is not hyaluronic acid, preferably not a polyglucosamine.

9. The polysaccharide for use of any one of embodiments 1 to 8, wherein the polysaccharide is selected from the group consisting of, unsubstituted amylopectin, substituted amylopectin, unsubstituted starch, substituted starch, unsubstituted maltodextrin, unsubstituted dextran, unsubstituted icodextrin, substituted maltodextrin, substituted dextran, and substituted icodextrin

10. The polysaccharide for use of any one of embodiments 1 to 9, wherein the polysaccharide comprises at least one substituted anhydroglucose unit, wherein the at least one anhydroglucose unit is preferably substituted with at least one substituent selected from the group consisting of glycosyl, alkyl, aryl, cycloalkyl, halogen, amino acid and -(alkyl-)$_n$-H with n = 1-6, preferably 1-3, and wherein the alkyl group in each repeating unit n may be the same or may differ from each other, and wherein in each group -(alkyl-O)$_n$-H present in the polysaccharide, n may be the same or may differ from each other.

11. The polysaccharide for use of any one of embodiments 1 to 10, wherein the polysaccharide comprises at least one substituted anhydroglucose unit being substituted with at least one group -(alkyl-O)$_n$-H with n = 1-6, preferably 1-3, and wherein the alkyl group in each repeating unit n may be the same or may differ from each other, and wherein in each group -(alkyl-O)$_n$-H present in the polysaccharide, n may be the same or may differ from each other.

12. The polysaccharide for use of any one of embodiments 1 to 11, wherein the polysaccharide comprises at least one substituted anhydroglucose unit being substituted with at least one group -(ethyl-O)$_n$-H with n = 1-6, preferably 1-3, and wherein the ethyl group in each repeating unit n may be the same or may differ from each other, and wherein in each group -(ethyl-O)$_n$-H present in the polysaccharide, n may be the same or may differ from each other.

13. The polysaccharide for use of any one of embodiments 1 to 12, wherein the polysaccharide is hydroxyalkyl starch.

14. The polysaccharide for use of any one of embodiments 1 to 13, wherein the polysaccharide is hydroxethyl starch.

15. The polysaccharide for use of any one of embodiments 1 to 12, wherein the polysaccharide is selected from the group consisting of dextran, hydroxyalkyl starch and icodextrin.

16. The polysaccharide for use of any one of embodiments 1 to 15, wherein the polysaccharide is dextran or HAS, more preferably HAS, most preferably HES, and wherein the polysaccharide is for postoperative administration or is to be administered postoperatively.

17. The polysaccharide for use of any one of embodiments 1 to 15, wherein the polysaccharide is dextran or HAS, more preferably HAS, most preferably HES, and wherein the polysaccharide is for intraoperative administration or is to be administered intraoperatively.

18. The polysaccharide for use of any one of embodiments 1 to 15, wherein the polysaccharide is icodextrin, dextran or HAS, more preferably dextran or HAS; more preferably HAS, most preferably HES, and wherein the polysaccharide is for preoperative administration or is to be administered preoperatively.

19. The polysaccharide for use of any one of embodiments 1 to 9, wherein the polysaccharide is icodextrin, and wherein the polysaccharide is for preoperative administration or is to be administered preoperatively.

20. The polysaccharide for use of any one of embodiments 10 to 14, wherein the polysaccharide has a molar substitution (MS) value in the range of from 0.1 to 3.

21. The polysaccharide for use of any one of embodiments 1 to 20 wherein the polysaccharide is osmotically active and/or biodegradable.

22. The polysaccharide for use of any one of embodiments 1 to 21, wherein the polysaccharide has an average molecular weight of 5 to 1200 kDa, preferably 70 to 800 kDa.

23. The polysaccharide for use of any one of embodiments 1 to 22, wherein said cancer forms a solid tumor.

24. The polysaccharide for use of any one of embodiments 1 to 23, wherein the cancer is ovarian cancer, ovarian carcinoma, stomach cancer, lung cancer, pancreas cancer, bladder cancer, liver cancer, colorectal cancer, or breast cancer.

25. The polysaccharide for use of any one of embodiments 1 to 24, wherein the polysaccharide is comprised in a pharmaceutically acceptable liquid solution.

26. The polysaccharide for use of embodiment 25, wherein the concentration of the polysaccharide in said composition is 1% (w/v) to 25% (w/v), preferably 2% (w/v) to 15% (w/v), more preferably 3% (w/v) to 12.5% (w/v), and most preferably 4% (w/v) to 10% (w/v), based on the total volume of the composition.

27. The polysaccharide for use of any one of embodiments 1 to 26, wherein metastasis and/or relapse in a body cavity of said subject, preferably the abdominal cavity, is prevented.

28. The polysaccharide for use of any one of embodiments 25 or 26, wherein the pharmaceutically acceptable liquid solution is an aqueous solution.

29. A composition, preferably a pharmaceutical composition, for use in preventing metastasis formation and/or relapse by administration to a body cavity of a subject afflicted with cancer, comprising a polysaccharide comprising optionally substituted monosaccharide units linked via alpha-glycosidic bonds.

30. The composition for use of embodiment 29, wherein the composition comprises a polysaccharide as the sole active ingredient preventing metastasis formation and/or relapse.

31. The composition for use of embodiment 29 or 30, wherein the polysaccharide is dextran or HAS, more preferably HAS, most preferably HES, and wherein the composition is for intraoperative administration or is to be administered intraoperatively.

32. The composition for use of embodiment 29 or 30, wherein the polysaccharide is dextran or HAS, more preferably HAS, most preferably HES, and wherein the composition is for postoperative administration or is to be administered postoperatively.

33. The composition for use of embodiment 29 or 30, wherein the polysaccharide is icodextrin, dextran or HAS, more preferably dextran or HAS; more preferably HAS, most preferably HES, and wherein the composition is for preoperative administration or is to be administered preoperatively.

34. The composition for use of embodiment 29 or 30, wherein the polysaccharide is icodextrin, and wherein the composition is for preoperative administration or is to be administered preoperatively.

35. Use of a polysaccharide, preferably of a polysaccharide as described in any one of embodiments 1 to 28, for the manufacture of a composition for preventing metastasis formation in a subject afflicted with cancer.

36. A method for preventing metastasis formation and/or relapse in a subject afflicted with cancer, comprising

a) administering a composition comprising a polysaccharide according to any one of embodiments 1 to 28 to a body cavity of said subject, and
b) thereby preventing metastasis formation in said subject.

37. The method for preventing metastasis formation in a subject afflicted with cancer of embodiment 36, wherein metastasis formation in a body cavity, preferably in the body cavity into which the polysaccharide was administered, is prevented.

38. The method for preventing metastasis formation in a body cavity of a subject afflicted with cancer of embodiment 36 or 37, wherein the cancer forms a solid tumor and wherein said method comprises the further step of removing of at least part of the primary tumor of said cancer after administering said aqueous solution comprising a polysaccharide in step a).

39. The method for preventing metastasis formation in a body cavity of a subject afflicted with cancer of any one of embodiments 36 to 38, wherein at least the primary tumor or a part thereof is removed by surgery.

40. The method for preventing metastasis formation in a body cavity of a subject afflicted with cancer of any one of embodiments 36 to 39, wherein at least said primary tumor is removed completely.

41. A kit comprising a polysaccharide, preferably a polysaccharide as defined in any one of embodiments 1 to 28, and a pharmaceutically acceptable means of solubilizing the same.

42. A device comprising a polysaccharide, preferably a polysaccharide as defined in any one of embodiments 1 to 28, and means for administering the same.

43. The device of embodiment 42, wherein the polysaccharide is comprised in an aqueous solution and wherein the means for administering are means for administering a liquid.

44. The polysaccharide for use of any one of embodiments 1 to 28, or the composition for use of embodiment 29 or 30, wherein the polysaccharide is for preoperative administration.

45. The polysaccharide for use of any one of embodiments 1 to 28, or the composition for use of embodiment 29 or 30, wherein the polysaccharide is for postoperative administration.

46. The polysaccharide for use of any one of embodiments 1 to 28, or the composition for use of embodiment 29 or 30, wherein the polysaccharide is for intraoperative administration.

47. The polysaccharide for use of any one of embodiments 1 to 28, or the composition for use of embodiment 29 or 30, wherein the subject is a human.

All references cited in this specification are herewith incorporated by reference with respect to their entire disclosure content and the disclosure content specifically mentioned in this specification.

**Figure Legends**

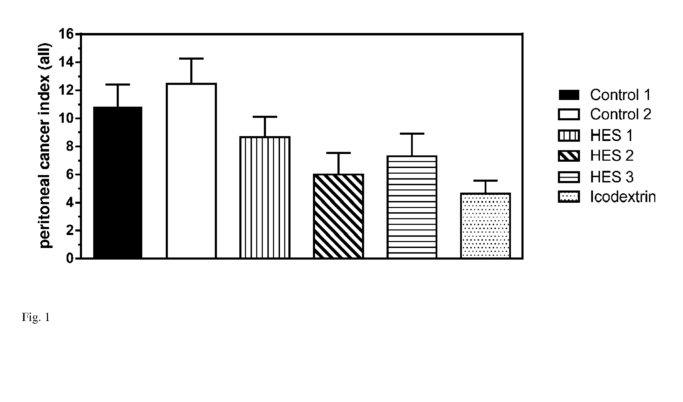

Figure 1 shows the development of the peritoneal cancer index (PCI; mean of all animals $\pm$ SEM) of mice inoculated with LS174T tumor cells as observed in example 1. On day 0, $2 \times 10^6$ LS174T cells were administered by intraperitoneal injection into the abdominal cavity of all BALB/c nude mice. Within 10 to 15 minutes after cell implantation, the mice were treated once intraperitoneally with various substances. The respective treatments are indicated by the following symbols. Black bar: 0.9 % isotonic saline (NaCl), indicated as "Control 1". White bar: Thelosan®, indicated as "Control 2". Vertical lines: Salinhes® (6 % HES 70/0.5), indicated as "HES 1". Diagonal lines: Voluven® 10 % (HES 130/0.4), indicated as "HES 2". Horizontal lines: Cardioplegische Perfusionslösung (HES 450/0.7), indicated as "HES 3". Dotted bar: Adept@, indicated as "Icodextrin".

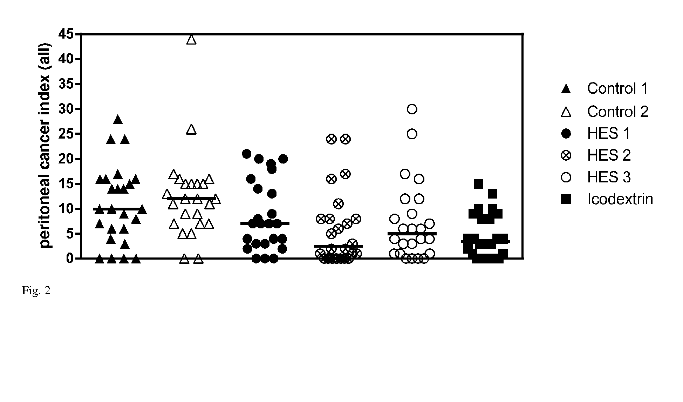

Figure 2 shows the development of peritoneal cancer index (PCI; single values of all animals; black line: median) of mice inoculated with LS174T tumor cells over time as observed in example 1. The treatments are indicated by the following symbols: the "▲" (black up-pointing triangle) is used when 0.9 % isotonic saline (NaCl) was administered to mice, indicated as "Control 1". The "∆" (white up-pointing triangle) is used when Thelosan® was administered to mice, indicated as "Control 2". The "●" (black circle) is used when Salinhes® (6 % HES 70/0.5) was administered to mice, indicated as "HES 1". The "⊗" (white, crossed circle) is used when Voluven® 10 % (HES 130/0.4) was administered to mice, indicated as "HES 2". The "○" (white circle) is used when Cardioplegische Perfusionslösung (HES 450/0.7) was administered to mice, indicated as "HES 3". The "■" (big black square) is used when Adept@ was administered to mice, indicated as "Icodextrin".

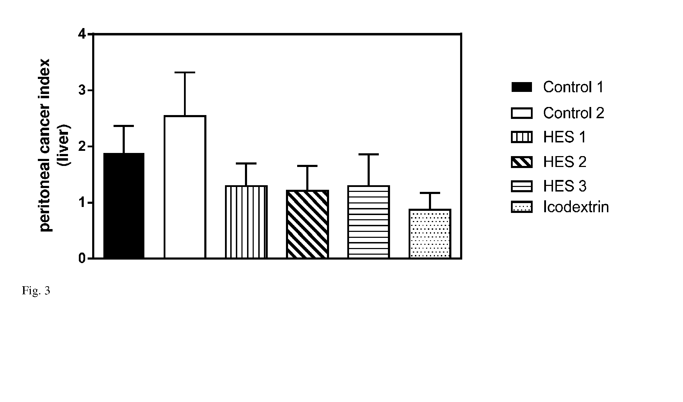

Figure 3 shows the development of the peritoneal cancer index (PCI; mean of all animals $\pm$ SEM) for the liver as observed in example 1. The respective treatments are indicated by the following symbols. Black bar: 0.9 % isotonic saline (NaCl), indicated as "Control 1". White bar: Thelosan®, indicated as "Control 2". Vertical lines: Salinhes® (6 % HES 70/0.5), indicated as "HES 1". Diagonal lines: Voluven® 10 % (HES 130/0.4), indicated as "HES 2". Horizontal lines: Cardioplegische Perfusionslösung (HES 450/0.7), indicated as "HES 3". Dotted bar: Adept@, indicated as "Icodextrin".

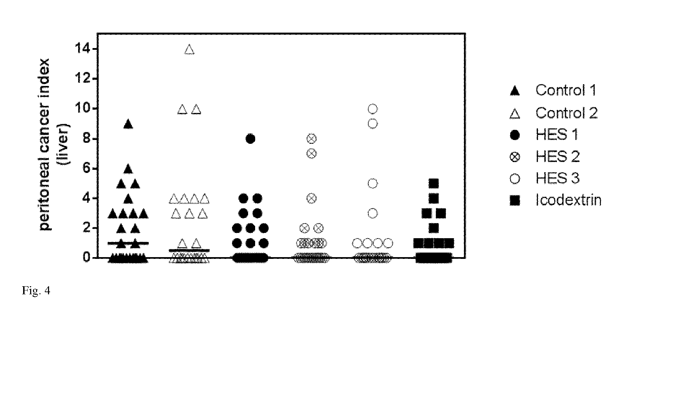

Figure 4 shows the development of peritoneal cancer index (PCI; single values of all animals; black line: median) for the liver as observed in example 1. The treatments are indicated by the following symbols: the "▲" (black up-pointing triangle) is used when 0.9 % isotonic saline (NaCl) was administered to mice, indicated as "Control 1". The "∆" (white up-pointing triangle) is used when Thelosan® was administered to mice, indicated as "Control 2". The "●" (black circle) is used when Salinhes® (6 % HES 70/0.5) was administered to mice, indicated as "HES 1". The "⊗" (white, crossed circle) is used when Voluven® 10 % (HES 130/0.4) was administered to mice, indicated as "HES 2". The "○" (white circle) is used when Cardioplegische Perfusionslösung (HES 450/0.7) was administered to mice, indicated as "HES 3". The "■" (big black square) is used when Adept@ was administered to mice, indicated as "Icodextrin".

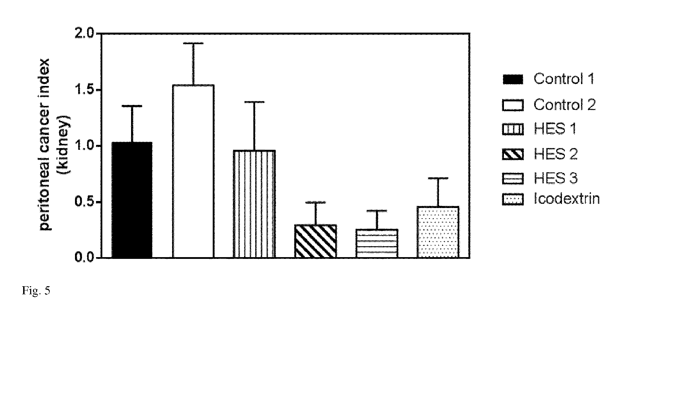

Figure 5 shows the development of the peritoneal cancer index (PCI; mean of all animals $\pm$ SEM) for the kidneys as observed in example 1. The respective treatments are indicated by the following symbols. Black bar: 0.9 % isotonic saline (NaCl), indicated as "Control 1". White bar: Thelosan®, indicated as "Control 2". Vertical lines: Salinhes® (6 % HES 70/0.5), indicated as "HES 1". Diagonal lines: Voluven® 10 % (HES 130/0.4), indicated as "HES 2". Horizontal lines: Cardioplegische Perfusionslösung (HES 450/0.7), indicated as "HES 3". Dotted bar: Adept@, indicated as "Icodextrin".

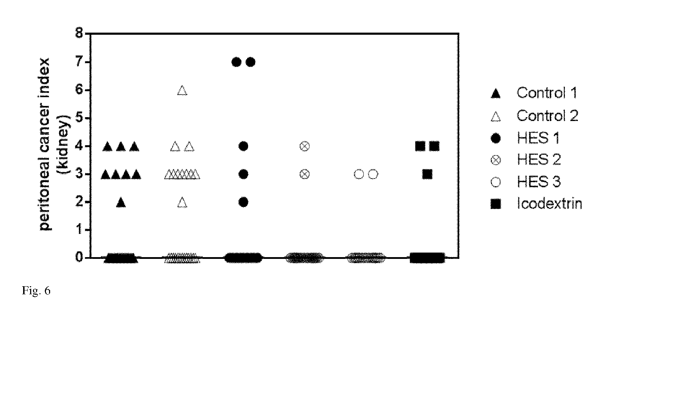

Figure 6 shows the development of peritoneal cancer index (PCI; single values of all animals; black line: median) for the kidney as observed in example 1. The treatments are indicated by the following symbols: the "▲" (black up-pointing triangle) is used when 0.9 % isotonic saline (NaCl) was administered to mice, indicated as "Control 1". The "∆" (white up-pointing triangle) is used when Thelosan® was administered to mice, indicated as "Control 2". The "●" (black circle) is used when Salinhes® (6 % HES 70/0.5) was administered to mice, indicated as "HES 1". The "⊗" (white, crossed circle) is used when Voluven® 10 % (HES 130/0.4) was administered to mice, indicated as "HES 2". The "○" (white circle) is used when Cardioplegische Perfusionslösung (HES 450/0.7) was administered to mice, indicated as "HES 3". The "■" (big black square) is used when Adept@ was administered to mice, indicated as "Icodextrin".

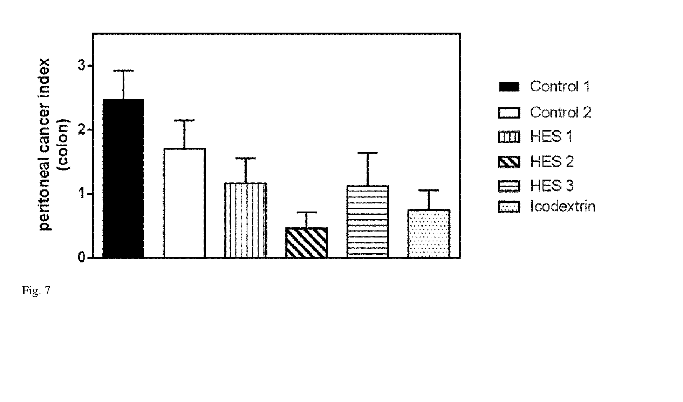

Figure 7 shows the development of the peritoneal cancer index (PCI; mean of all animals $\pm$ SEM) for the colon as observed in example 1. The respective treatments are indicated by the following symbols. Black bar: 0.9 % isotonic saline (NaCl), indicated as "Control 1". White bar: Thelosan®, indicated as "Control 2". Vertical lines: Salinhes® (6 % HES 70/0.5), indicated as "HES 1". Diagonal lines: Voluven® 10 % (HES 130/0.4), indicated as "HES 2". Horizontal lines: Cardioplegische Perfusionslösung (HES 450/0.7), indicated as "HES 3". Dotted bar: Adept@, indicated as "Icodextrin".

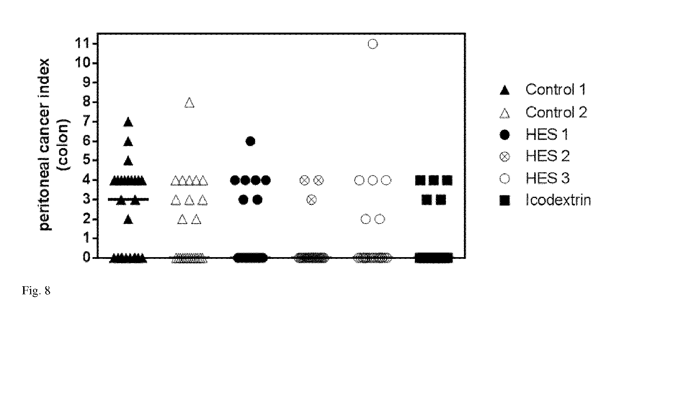

Figure 8 shows the development of peritoneal cancer index (PCI; single values of all animals; black line: median) for the colon as observed in example 1. The treatments are indicated by the following symbols: the "▲" (black up-pointing triangle) is used when 0.9 % isotonic saline (NaCl) was administered to mice, indicated as "Control 1". The "∆" (white up-pointing triangle) is used when Thelosan® was administered to mice, indicated as "Control 2". The "●" (black circle) is used when Salinhes® (6 % HES 70/0.5) was administered to mice, indicated as "HES 1". The "⊗" (white, crossed circle) is used when Voluven® 10 % (HES 130/0.4) was administered to mice, indicated as "HES 2". The "○" (white circle) is used when Cardioplegische Perfusionslösung (HES 450/0.7) was administered to mice, indicated as "HES 3". The "■" (big black square) is used when Adept@ was administered to mice, indicated as "Icodextrin".

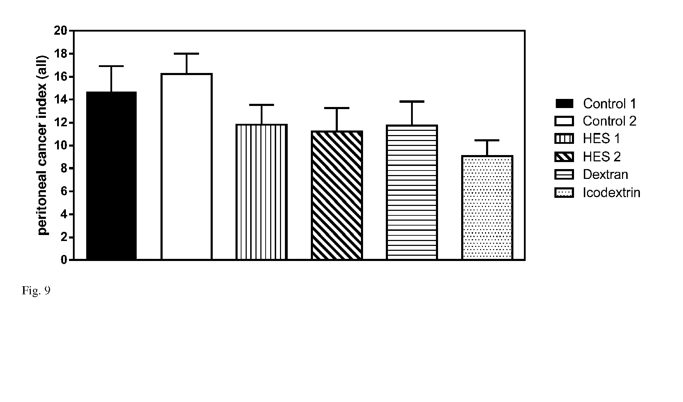

Figure 9 shows the development of the peritoneal cancer index (PCI; mean of all animals ± SEM) of mice inoculated with LS174T tumor cells as observed in example 2. On day 0, $2 \times 10^6$ LS174T cells were administered by intraperitoneal injection into the abdominal cavity of all BALB/c nude mice. Within 10 to 15 minutes after cell implantation, the mice were treated once intraperitoneally with various substances. The respective treatments are indicated by the following symbols. Black bar: untreated, indicated as "Control 1". White bar: 0.9 % isotonic saline (NaCl), indicated as "Control 2". Vertical lines: Voluven 6 % (HES 130/0.4), indicated as "HES 1". Diagonal lines: Voluven® 10 % (HES 130/0.4), indicated as "HES 2". Horizontal lines: 10 % Dextran 40, indicated as "Dextran". Dotted bar: Adept@, indicated as "Icodextrin".

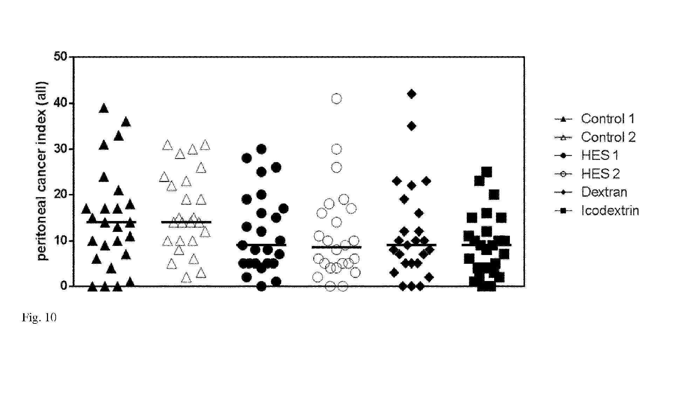

Figure 10 shows the development of peritoneal cancer index (PCI; single values of all animals; black line: median) of mice inoculated with LS174-T tumor cells over time as observed in example 2. The treatments are indicated by the following symbols: the "▲" (black up-pointing triangle) is used for untreated mice, indicated as "Control 1". The "△" (white up-pointing triangle) is used when 0.9 % isotonic saline (NaCl) was administered to mice, indicated as "Control 2". The "●" (black circle) is used when Voluven® 6 % (HES 130/0.4) was administered to mice, indicated as "HES 1". The "○" (white circle) is used when Voluven® 10 % (HES 130/0.4) was administered to mice, indicated as "HES 2". The "♦" (black diamond) is used when 10 % Dextran 40 was administered to mice, indicated as "Dextran". The "■" (big black square) is used when Adept@ was administered to mice, indicated as "Icodextrin".

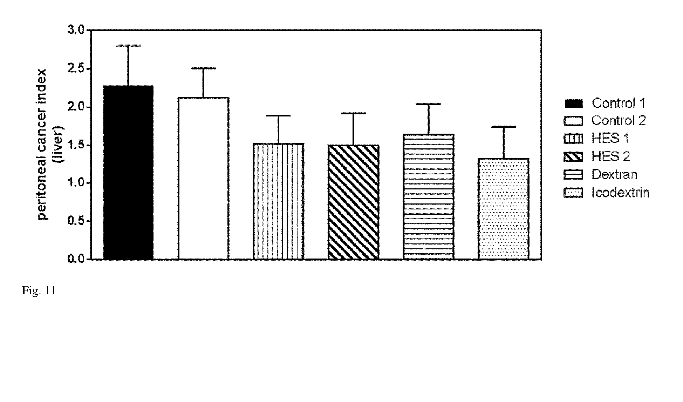

Figure 11 shows the development of the peritoneal cancer index (PCI; mean of all animals ± SEM) for the liver as observed in example 2. The respective treatments are indicated by the following symbols. Black bar: untreated, indicated as "Control 1". White bar: 0.9 % isotonic saline (NaCl), indicated as "Control 2". Vertical lines: Voluven 6 % (HES 130/0.4), indicated as "HES 1". Diagonal lines: Voluven® 10 % (HES 130/0.4), indicated as "HES 2". Horizontal lines: 10 % Dextran 40, indicated as "Dextran". Dotted bar: Adept@, indicated as "Icodextrin".

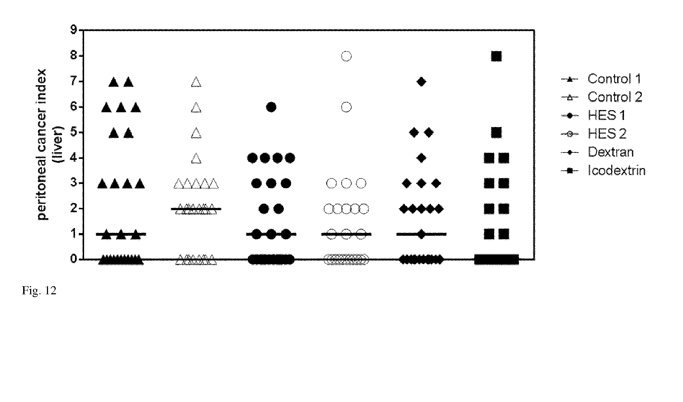

Figure 12 shows the development of peritoneal cancer index (PCI; single values of all animals; black line: median) for the liver as observed in example 2. The treatments are indicated by the following symbols: the "▲" (black up-pointing triangle) is used for untreated mice, indicated as "Control 1". The "△" (white up-pointing triangle) is used when 0.9 % isotonic saline (NaCl) was administered to mice, indicated as "Control 2". The "●" (black circle) is used when Voluven® 6 % (HES 130/0.4) was administered to mice, indicated as "HES 1". The "○" (white circle) is used when Voluven® 10 % (HES 130/0.4) was administered to mice, indicated as "HES 2". The "♦" (black diamond) is used when 10 % Dextran 40 was administered to mice, indicated as "Dextran". The "■" (big black square) is used when Adept@ was administered to mice, indicated as "Icodextrin".

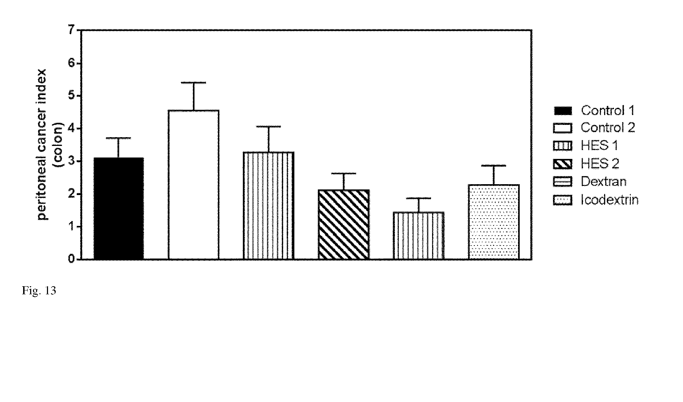

Figure 13 shows the development of the peritoneal cancer index (PCI; mean of all animals ± SEM) for the colon as observed in example 2. The respective treatments are indicated by the following symbols. Black bar: untreated, indicated as "Control 1". White bar: 0.9 % isotonic saline (NaCl), indicated as "Control 2". Vertical lines: Voluven 6 % (HES 130/0.4), indicated as "HES 1". Diagonal lines: Voluven® 10 % (HES 130/0.4), indicated as "HES 2". Horizontal lines: 10 % Dextran 40, indicated as "Dextran". Dotted bar: Adept@, indicated as "Icodextrin".

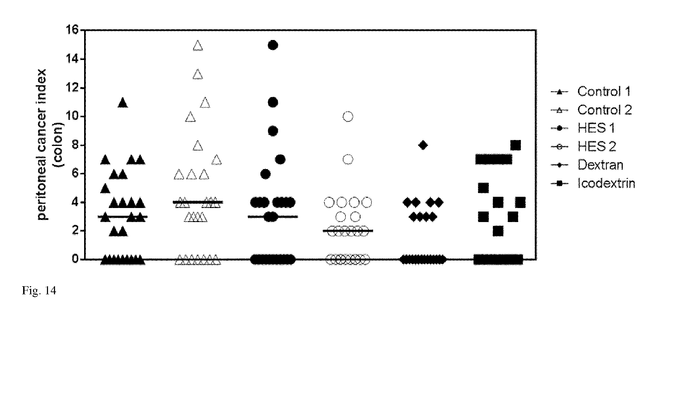

Figure 14 shows the development of peritoneal cancer index (PCI; single values of all animals; black line: median) for the colon as observed in example 2. The treatments are indicated by the following symbols: the "▲" (black up-pointing triangle) is used for untreated mice, indicated as "Control 1". The "△" (white up-pointing triangle) is used when 0.9 % isotonic saline (NaCl) was administered to mice, indicated as "Control 2". The "●" (black circle) is used when Voluven® 6 % (HES 130/0.4) was administered to mice, indicated as "HES 1". The "○" (white circle) is used when Voluven® 10 % (HES 130/0.4) was administered to mice, indicated as "HES 2". The "♦" (black diamond) is used when 10 % Dextran 40 was administered to mice, indicated as "Dextran". The "■" (big black square) is used when Adept@ was administered to mice, indicated as "Icodextrin".

The following Examples shall merely illustrate the invention. They shall not be construed, whatsoever, to limit the scope of the invention.

**Example 1:**

Summary: Adult female BALB/c nude mice were treated with a single i.p. injection of saline, Thelosan®, Salinhes® fluid solution 6%, Voluven® 10%, Cardioplegische Perfusionslösung ("Cardioplegic perfusion solution" - see below) or Adept® after inoculation with human colon carcinoma cells LS 174T to determine tumor cell growth and body weight over the course of the experiment.

Substances:

Saline (0.9% NaCl) (Lot 120148091, Fresenius Kabi Deutschland GmbH, Bad Homburg, Germany) and Thelosan (Natriumhyaluronate 800 mg/L, chondroitin sulphate 800 mg/L, NaCl 8.3 g/L in aqua ad iniectabilia) (Lot 20EBD010, Fresenius Kabi Deutschland GmbH, Bad Homburg, Germany) were used as Control 1 and Control 2. The hydroxyethyl starch (HES) containing test items Salinhes® fluid solution 6% (Poly(O-2-hydroxyethyl)starch (HES 70/0.5) 60 g/L, NaCl 9 g/L) (Lot 90FIS521), Voluven® 10% (Poly(O-2-hydroxyethyl)starch (HES 130/0.4) 100 g/L, NaCl 9 g/L) (Lot 14FC3308) and Cardioplegische Perfusionslösung (Poly(O-2-hydroxyethyl)starch (HES 450/0.7) 60 g/L, magnesium-DL-hydrogenaspartate 4 H2O 0.721 g/L, Procainhydrochlorid 1.091 g/L, calcium chloride 2 H2O 0.074 g/L, sodium chloride 1.461 g/L, potassium chloride 0.373 g/L, glucose-monohydrate 1.982 g/L, mannitol 36.44 g/L, other constituents: hydrochloric acid, sodium hydroxide, water for injection) (Lot 16FA0199) were obtained from Fresenius Kabi Deutschland GmbH (Bad Homburg, Germany) as ready-to-use products. Adept® (Icodextrin 40 g/L, sodium chloride 5.4 g/L, sodium lactate 4.5 g/L, calcium chloride 257 mg/L, magnesium chloride 61 mg/L) (Lot 11892004) was obtained from Baxter Deutschland GmbH (Unterschleisheim, Germany) as ready-to-use product. All solutions were stored at room temperature (<25°C) until use. All solutions were injected under sterile conditions.

Animals:

Adult female BALB/c nude mice (strain CAnN.Cg-Foxn1nu/Crl) (Charles River GmbH, Sulzfeld, Germany) were used in the study. At the start of experiment they were 5-6 weeks of age and had a median body weight between 16 and 18 g.

All mice were maintained under strictly controlled and standardized barrier conditions. They were housed - maximum four mice/cage - in individually ventilated cages under following environmental conditions: 22+/-3°C room temperature, 30 - 70% relative humidity, 12 hours artificial fluorescent light / 12 hours dark. They received autoclaved food and bedding (Ssniff, Soest, Germany) and autoclaved community tap water ad libitum.

Carcinomatosis model:

The study consisted of 6 experimental groups each containing either 25 (Group 1) or 24 (Groups 2-6) female BALB/c nude mice. On day 0, $2 \times 10^6$ LS174T cells in 300$\mu$l PBS were administered by intraperitoneal injection into the abdominal cavity of all BALB/c nude mice (Groups 1-6). Freshly prepared cell suspensions were used for each round of implantation, in which 4 animals each of Groups 1-6 were implanted. For the implantation of 24 animals per group, 6 rounds of implantation with freshly prepared cell suspensions for 24 animals (Groups 1-6) were needed (cells for one additional animal of Group 1 were taken into account in the last round). Within 10 to 15 minutes after cell implantation, animals were treated once intraperitoneally with different starch solutions and Thelosan® (Groups 2-5). Animals of Group 6 received Adept®. All solutions were administered as supplied, each in a volume of 500 $\mu$l per mouse. Animals of Group 1 received 500 $\mu$l saline solution (see Table 1).

Table 1

| Group | Treatment | Administration volume | Route of application | Animal Number |
|---|---|---|---|---|
| 1 | Saline | 500 $\mu$l/mouse | i.p. | 25 |
| 2 | Thelosan® | 500 $\mu$l/mouse | i.p. | 24 |
| 3 | Salinhes® fluid solution 6% | 500 $\mu$l/mouse | i.p. | 24 |
| 4 | Voluven® 10% | 500 $\mu$l/mouse | i.p. | 24 |
| 5 | Cardioplegische Perfusionslösung | 500 $\mu$l/mouse | i.p. | 24 |
| 6 | Adept® | 500 $\mu$l/mouse | i.p. | 24 |

Animal weights were taken every other day (Monday, Wednesday and Friday). Animal behaviour was monitored daily.

During the course of the study, several animals out of all study groups were sacrificed due to ethical reasons (ascites, paresis, swelling of the abdominal wall) ahead of schedule and a necropsy performed. On study day 43, the study was terminated due to ethical reasons, all remaining animals sacrificed and a necropsy performed. At necropsy, all animals were weighed and killed by cervical dislocation. Animals were macroscopically inspected and a quantification of visible tumours was performed by calculating the peritoneal cancer index (PCI).

For this purpose, all tumors of the abdominal cave were categorized via eleven different regions of interest (see Table 2 below) and classified according to the Lesion-Size Score into LS-0 to LS-4 using the tumour diameters, listed in Table 2. Then the number of tumors within the different regions of interest for each Lesion-Size were added up and multiplied with the corresponding factor 0, 1, 2, 3 or 4 for LS-0, LS-1, LS-2, LS-3 and LS-4, respectively, to obtain the Lesion-Size specific PCI values $PCI_{LS0}$ to $PCI_{LS4}$. Finally, these five results were added up in order to get the total Peritoneal Cancer Index ($PCI_{total}$).

Additionally, organ-specific PCI values were calculated for each group. For this purpose, individual PCI values for each region of interest were calculated for each animal as described above, obtaining the organ-specific PCI values $PCI_{RI1}$ to $PCI_{RI11}$. Finally, for each region of interest, $PCI_{RI}$ values for all animals per group were added up and mean and median values determined.

Table 2: Peritoneal Cancer Index (PCI): evaluation scheme

| Regions of interest (RI) | | Lesion-Size Score (LS) | | | | | RI-specific PCI values |
|---|---|---|---|---|---|---|---|
| | | LS-0 | LS-1 | LS-2 | LS-3 | LS-4 | |
| | | No visible tumours | <2mm tumour diameter | 2-5mm tumour diameter | 5-10mm tumour diameter | >10mm tumour diameter | |
| 1 | Right peritoneum | | | | | | $PCI_{RI1}$ |
| 2 | Left peritoneum | | | | | | $PCI_{RI2}$ |
| 3 | Stomach | | | | | | $PCI_{RI3}$ |
| 4 | Kidney | | | | | | $PCI_{RI4}$ |
| 5 | Intestine | | | | | | $PCI_{RI5}$ |
| 6 | Caecum | | | | | | $PCI_{RI6}$ |
| 7 | Colon | | | | | | $PCI_{RI7}$ |
| 8 | Liver | | | | | | $PCI_{RI8}$ |
| 9 | Spleen | | | | | | $PCI_{RI9}$ |
| 10 | Diaphragm | | | | | | $PCI_{RI10}$ |
| 11 | Mesentery | | | | | | $PCI_{RI11}$ |
| Lesion-Size specific PCI values | | $PCI_{LS0}$ | $PCI_{LS1}$ | $PCI_{LS2}$ | $PCI_{LS3}$ | $PCI_{LS4}$ | $\Sigma=PCI_{total}$ |

Statistical evaluation:

Animal weights, PCI total values per group as well as organ-specific PCI values were analysed using descriptive data analysis (Mean with SEM; Median). In addition, all single values are shown as well over all samples and organ-specific (single values and median). All data analyses were performed using GraphPad Prism 5 from GraphPad Software, Inc., San Diego, USA.

Results:

Hydroxyethyl starch and Icodextrin caused a clear reduction of the peritoneal cancer index compared to the Control groups (Figs. 1 and 2). This effect was observed especially in liver (Figs. 3 and 4), kidney (Fig. 5 and 6), and colon (Figs. 7 and 8). No substance related toxicity was detected since the animal weight development was similar for all groups (not shown).

**Example 2**

Summary: Adult female BALB/c nude mice were treated with a single i.p. injection of saline, Voluven® 6%, Voluven® 10%, Dextran 40 10% or Adept® after inoculation with human colon carcinoma cells LS 147T to determine tumor cell growth and body weight over the course of the experiment in comparison to an untreated Control group.

Substances:

Saline (0.9% NaCl) (Lot 120148091, B. Braun Melsungen AG, Melsungen, Germany) was used as Control 2. The hydroxyethyl starch (HES) containing test items Voluven® 6% Poly(O-2-hydroxyethyl)starch (HES 130/0.4) 60 g/L, NaCl 9 g/L) (Lot 14EL3310) and Voluven® 10% (Poly(O-2-hydroxyethyl)starch (HES 130/0.4) 100 g/L, NaCl 9 g/L) (Lot 14FC3308) were obtained from Fresenius Kabi Deutschland GmbH (Bad Homburg, Germany) as ready-to-use products. Dextran 40 10% (Polyglucose 100 g/L, NaCl 9 g/L) (Lot 2881143432) was obtained from AlleMan Pharma GmbH (Rimbach, Germany) and Adept® (Icodextrin 40 g/L, sodium chloride 5.4 g/L, sodium lactate 4.5 g/L, calcium chloride 257 mg/L, magnesium chloride 61 mg/L) (Lot 11892004) was obtained from Baxter Deutschland GmbH (Unterschleißheim, Germany) as ready-to-use product. All solutions were stored at room temperature (<25°C) until use. All solutions were injected under sterile conditions.

Animals:

Adult female BALB/c nude mice (strain CAnN.Cg-Foxn1nu/Crl) (Charles River GmbH, Sulzfeld, Germany) were used in the study. At the start of experiment they were 5-6 weeks of age and had a median body weight between 16 and 20 g.
All mice were maintained under strictly controlled and standardized barrier conditions. They were housed - maximum four mice/cage - in individually ventilated cages under following environmental conditions: 22+/-3°C room temperature, 30 - 70% relative humidity, 12 hours artificial fluorescent light / 12 hours dark. They received autoclaved food and bedding (Ssniff, Soest, Germany) and autoclaved community tap water ad libitum.

Carcinomatosis model:

The study consisted of 6 experimental groups each containing 25 female BALB/c nude mice. On day 0, $2\times10^6$ LS174T cells in 300µl PBS were administered by intraperitoneal injection into the abdominal cavity of all BALB/c nude mice (Groups 1-6). Freshly prepared cell suspensions were used for each round of implantation, in which 4 animals each of Groups 1-6 were implanted. For the implantation of 25 animals per group, 6 rounds of implantation with freshly prepared cell suspensions for 25 animals (Groups 1-6) were needed (cells for one additional animal of Group 1 were taken into account in the last round). Within 10 to 15 minutes after cell implantation, animals were treated once intraperitoneally with Voluven® 6%, Voluven® 10%, Dextran 40 10% and Adept® (Groups 3-6). All solutions were administered as supplied, each in a volume of 500 µl per mouse. Animals of Group 1 remained untreated and animals of Group 2 received 500 µl saline solution (see Table 3).

Table 3

| Group | Treatment | Administration volume | Route of application | Animal Number |
|---|---|---|---|---|
| 1 | Untreated | - | i.p. | 25 |
| 2 | Saline | 500 µl/mouse | i.p. | 25 |
| 3 | Voluven® 6% | 500 µl/mouse | i.p. | 25 |
| 4 | Voluven® 10% | 500 µl/mouse | i.p. | 25 |
| 5 | Dextran 40 10% | 500 µl/mouse | i.p. | 25 |
| 6 | Adept® | 500 µl/mouse | i.p. | 25 |

Animal weights were taken every other day (Monday, Wednesday and Friday). Animal behaviour was monitored daily.
During the course of the study, several animals out of all study groups were sacrificed due to ethical reasons (ascites, paresis, swelling of the abdominal wall) ahead of schedule and a necropsy performed. On study day 34, the study was terminated due to ethical reasons, all remaining animals sacrificed and a necropsy performed. At necropsy, all animals were weighed and killed by cervical dislocation. Animals were macroscopically inspected and a quantification of visible tumours was performed by calculating the peritoneal cancer index (PCI).
For this purpose, all tumors of the abdominal cave were categorized via eleven different regions of interest (see Table 2 above) and classified according to the Lesion-Size Score into LS-0 to LS-4 using the tumour diameters, listed in Table 2. Then the number of tumors within the different regions of interest for each Lesion-Size were added up and multiplied with the corresponding factor 0, 1, 2, 3 or 4 for LS-0, LS-1, LS-2, LS-3 and LS-4, respectively, to obtain the Lesion-Size specific PCI values $PCI_{LS0}$ to $PCI_{LS4}$. Finally, these five results were added up in order to get the total Peritoneal Cancer Index ($PCI_{total}$).

Additionally, organ-specific PCI values were calculated for each group. For this purpose, individual PCI values for each region of interest were calculated for each animal as described above, obtaining the organ-specific PCI values $PCI_{RI1}$ to $PCI_{RI11}$. Finally, for each region of interest, $PCI_{RI}$ values for all animals per group were added up and mean and median values determined.

Statistical evaluation:

Animal weights, PCI total values per group as well as organ-specific PCI values were analysed using descriptive data analysis (Mean with SEM; Median). In addition, all single values are shown as well over all samples and organ-specific (single values and median). All data analyses were performed using GraphPad Prism 5 from GraphPad Software, Inc., San Diego, USA.

Results:

Hydroxyethyl starch, Dextran 40, and Icodextrin caused a clear reduction of the peritoneal cancer index compared to the Control groups (Figs 9 and 10). This effect was observed especially in liver (Figs. 11 and 12) and colon (Figs. 13 and 14). No substance related toxicity was detected since the animal weight development was similar in all groups.

**Claims**

1. A polysaccharide comprising, optionally substituted, monosaccharide units linked via alpha-glycosidic bonds for use in preventing metastasis formation and/or relapse by administration to a body cavity of a subject afflicted with cancer.

2. The polysaccharide for use of claim 1, wherein the polysaccharide is for postoperative administration, for intraoperative administration, and/or for preoperative administration.

3. The polysaccharide for use of claim 1 or 2, wherein the polysaccharide comprises glycosidically linked anhydroglucose units, preferably comprises alpha-1,4-glycosidically linked anhydroglucose units and alpha-1,6-glycosidically linked anhydroglucose units, wherein the anhydroglucose units are optionally substituted.

4. The polysaccharide for use of any one of claims 1 to 3, wherein the polysaccharide further comprises at least one hydroxyalkyl group.

5. The polysaccharide use of any one of claims 1 to 4, wherein the polysaccharide is hydroxyalkyl starch, preferably hydroxethyl starch.

6. The polysaccharide for use of any one of claims 1 to 5, wherein said cancer forms a solid tumor.

7. The polysaccharide for use of any one of claims 1 to 6, wherein the cancer is ovarian cancer, ovarian carcinoma, stomach cancer, lung cancer, pancreas cancer, bladder cancer, liver cancer, colorectal cancer, or breast cancer.

8. The polysaccharide for use of any one of claims 1 to 7, wherein said polysaccharide is comprised in a pharmaceutically acceptable solution, preferably an aqueous solution.

9. The polysaccharide for use of claim 8, wherein the concentration of the polysaccharide in said pharmaceutically acceptable solution is 1% (w/v) to 25% (w/v), preferably 2% (w/v) to 15% (w/v), more preferably 3% (w/v) to 12.5% (w/v), and most preferably 4% (w/v) to 10% (w/v).

10. The polysaccharide for use of any one of claims 1 or 9, wherein metastasis and/or relapse in a body cavity of said subject, preferably the abdominal cavity, is prevented.

11. The polysaccharide for use of any one of claims 1 to 10, wherein the polysaccharide has an average molecular weight of 5 to 1200 kDa, preferably 13 to 800 kDa.

**12.** The polysaccharide for use of claim 4 or 5, wherein the polysaccharide has a molar substitution (MS) value in the range of from 0.1 to 3.

**13.** A composition, preferably a pharmaceutical composition, for use in preventing metastasis formation and/or relapse by administration to a body cavity of a subject afflicted with cancer, comprising a polysaccharide comprising, optionally substituted, monosaccharide units linked via alpha-glycosidic bonds.

**14.** The composition for use of claim 13, wherein the composition comprises a polysaccharide as the sole ingredient preventing metastasis formation and/or relapse.

**15.** A kit comprising a composition comprising a polysaccharide according to any one of claims 1 to 14 and a pharmaceutically acceptable means of solubilizing the same.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

EP 2 832 360 A1

Fig. 14

Europäisches
Patentamt

European
Patent Office

Office européen
des brevets

# EUROPEAN SEARCH REPORT

**Application Number**

EP 13 17 8566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JACOBI C A ET AL: "New therapeutic strategies to avoid intra- and extraperitoneal metastases during laparoscopy: results of a tumor model in the rat", DIGESTIVE SURGERY, S. KARGER AG, CH, vol. 16, no. 5, 1 January 1999 (1999-01-01), pages 393-399, XP008166387, ISSN: 0253-4886, DOI: 10.1159/000018754 * abstract * * page 395, column 1, paragraph 2-5 * * page 399, paragraph 2 * ----- | 1-14 | INV. A61K31/716 A61K31/718 A61K31/721 A61P35/04 |
| Y | WO 2010/096466 A2 (NOVELMED THERAPEUTICS INC [US]; BANSAL REKHA [US]) 26 August 2010 (2010-08-26) * claims 1,3,29-31 * ----- | 1-14 | |

TECHNICAL FIELDS
SEARCHED (IPC)

A61K

~~The present search report has been drawn up for all claims~~

| Place of search | Date of completion of the search | Examiner |
|---|---|---|
| The Hague | 2 January 2014 | Strack, Eberhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

**Europäisches Patentamt**
**European Patent Office**
**Office européen des brevets**

Application Number

EP 13 17 8566

---

### CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

### LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-14(partially)

    A polysaccharide comprising, optionally substituted, monosaccharide units linked via alpha-glycosidic bonds for use in preventing metastasis formation and/or relapse by administration to a body cavity of a subject afflicted with cancer, wherein the cancer is ovarian cancer or ovarian carcinoma

    ---

2. claims: 1-14(partially)

    A polysaccharide comprising, optionally substituted, monosaccharide units linked via alpha-glycosidic bonds for use in preventing metastasis formation and/or relapse by administration to a body cavity of a subject afflicted with cancer, wherein the cancer is stomach cancer

    ---

3. claims: 1-14(partially)

    A polysaccharide comprising, optionally substituted, monosaccharide units linked via alpha-glycosidic bonds for use in preventing metastasis formation and/or relapse by administration to a body cavity of a subject afflicted with cancer, wherein the cancer is lung cancer

    ---

4. claims: 1-14(partially)

    A polysaccharide comprising, optionally substituted, monosaccharide units linked via alpha-glycosidic bonds for use in preventing metastasis formation and/or relapse by administration to a body cavity of a subject afflicted with cancer, wherein the cancer is pancreas cancer

    ---

5. claims: 1-14(partially)

    A polysaccharide comprising, optionally substituted, monosaccharide units linked via alpha-glycosidic bonds for use in preventing metastasis formation and/or relapse by administration to a body cavity of a subject afflicted with cancer, wherein the cancer is bladder cancer

    ---

6. claims: 1-14(partially)

    A polysaccharide comprising, optionally substituted, monosaccharide units linked via alpha-glycosidic bonds for use in preventing metastasis formation and/or relapse by

Europäisches
Patentamt

European
Patent Office

Office européen
des brevets

**LACK OF UNITY OF INVENTION**
**SHEET B**

Application Number

EP 13 17 8566

The Search Division considers that the present European patent application does not comply with the requirements of unity of invention and relates to several inventions or groups of inventions, namely:

administration to a body cavity of a subject afflicted with cancer, wherein the cancer is liver cancer
---

7. claims: 1-14(partially)

A polysaccharide comprising, optionally substituted, monosaccharide units linked via alpha-glycosidic bonds for use in preventing metastasis formation and/or relapse by administration to a body cavity of a subject afflicted with cancer, wherein the cancer is colorectal cancer
---

8. claims: 1-14(partially)

A polysaccharide comprising, optionally substituted, monosaccharide units linked via alpha-glycosidic bonds for use in preventing metastasis formation and/or relapse by administration to a body cavity of a subject afflicted with cancer, wherein the cancer is breast cancer
---

9. claim: 15

A kit comprising a composition comprising a polysaccharide according to any one of claims 1 to 14 and a pharmaceutically acceptable means of solubilizing the same.
---

## ANNEX TO THE EUROPEAN SEARCH REPORT
## ON EUROPEAN PATENT APPLICATION NO.

EP 13 17 8566

This annex lists the patent family members relating to the patent documents cited in the above-mentioned European search report.
The members are as contained in the European Patent Office EDP file on
The European Patent Office is in no way liable for these particulars which are merely given for the purpose of information.

02-01-2014

| Patent document cited in search report | Publication date | Patent family member(s) | | Publication date |
|---|---|---|---|---|
| WO 2010096466 A2 | 26-08-2010 | CA | 2753698 A1 | 26-08-2010 |
| | | CN | 102405050 A | 04-04-2012 |
| | | EP | 2398482 A2 | 28-12-2011 |
| | | US | 2012040928 A1 | 16-02-2012 |
| | | WO | 2010096466 A2 | 26-08-2010 |

# EP 2 832 360 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007084661 A
- US 4207312 A
- WO 2004030613 A
- DE 4023788 A1
- US 6207654 B
- US 5807833 A
- WO 2012004007 A1

### Non-patent literature cited in the description

- **SOMMERMEYER et al.** *Krankenhauspharmazie,* 1987, vol. 8 (8), 271-278
- **WEIDLER et al.** *Arzneimittelforschung/Drug Research,* 1991, vol. 41, 494-498
- **A. WEITBERG.** *J Exp Clin Cancer Res,* 2008, vol. 27, 40
- **CHAN et al.** *J Hematol Oncol,* 2009, vol. 2, 25
- **MOHAMED et al.** *EJSO,* 2003, vol. 29, 261
- *Surg Oncol Clin N Am,* 2003, vol. 12, 813
- **ROCCONI et al.** *Gynecologic Oncology,* 2006, vol. 103, 985
- **VAN DEN TOL et al.** *Surgery,* 2005, vol. 137 (3), 348
- **I. BEKES.** Adhäsions- und Nidationsprophylaxe nach i.p. Implantation von SCOV.ip-Zellen in SCID-Mäuse mittels Icodextrin, Hyaluronsäure und physiologischer NaCl-Lösung. *Dissertation an der Medizinischen Fakultät der RWTH Aachen,* 2008
- **W.M. KULICKE ; U. KAISER ; D. SCHWENGERS ; R. LEMMES.** *Starch,* 1991, vol. 43 (10), 392-396
- **SOMMERMEYER et al.** *Krankenhauspharmazie,* 1987, vol. 8 (08), 271-278
- **YING-CHE LEE et al.** *Anal. Chem.,* 1983, vol. 55, 334-338
- **K. L. HODGES et al.** *Anal. Chem.,* 1979, vol. 51, 2171
- **SOMMERMEYER et al.** *Chromatographia,* 1988, vol. 25, 167-168
- **C. JUNGHEINRICH et al.** *Clin. Pharmacokin.,* 2005, vol. 44 (7), 681-699
- **J.-M. MISHLER IV.** Pharmacology of hydroxyethyl starches. Oxford Medical Publications, 2002, vol. 20, 1-30
- British Pharmacopoeia. *Appendix V: N. Osmolality,* 2012, vol. V